# FASCICULE DE BREVET EUROPEEN

(11) **EP 3 393 444 B1**
(45) Date de publication et mention de la délivrance du brevet: **30.08.2023**
(21) Numéro de dépôt: 16812977.3
(22) Date de dépôt: 21.12.2016
(51) Int. Cl.: A61K 8/49, A61Q 17/00, A61Q 17/04, A61Q 19/00, A61Q 19/06, A61Q 19/08, A61K 8/97, A61K 36/185, A61P 17/16, A61P 29/00, A61K 31/192, A61K 31/353

(54) **EXTRAIT DE GRAINES DE PASSIFLORE ET COMPOSITIONS COSMETIQUES, PHARMACEUTIQUES OU DERMATOLOGIQUES LE COMPRENANT**
PASSIONSBLUMEN-EXTRAKT UND KOSMETISCHEN, PHARMAZEUTISCHEN ODER DERMATOLOGISCHEN ZUSAMMENSETZUNGEN, DIE DIESE UMFASSEN.
PASSIFLORE SEED EXTRACTS AND COSMETIC, PHARMACEUTICAL OR DERMATOLOGICAL COMPOSITIONS THEREOF.

(30) Priorité: 21.12.2015 FR 1562949
(43) Date de publication de la demande: 31.10.2018
(73) Titulaire: Laboratoires Expanscience, 92048 Paris La Défense Cedex (FR)
(72) Inventeur: LECLERE-BIENFAIT, Sophie, 28100 Dreux (FR); BREDIF, Stéphanie, 28210 Croisilles (FR)
(74) Mandataire: Regimbeau
(86) Numéro de dépôt international: PCT/EP2016/082216
(87) Numéro de publication internationale: WO 2017/108978

(56) Documents cités:
- FR-A1- 3 010 906
- KR-A- 20150 064 314
- NATTAYA LOURITH ET AL: "Antioxidant Activities and Phenolics of Passiflora edulis Seed Recovered from Juice Production Residue", JOURNAL OF OLEO SCIENCE, vol. 62, no. 4, 1 janvier 2013 (2013-01-01), pages 235-240, XP055286455, JP ISSN: 1345-8957, DOI: 10.5650/jos.62.235
- Yuko Matsui ET AL: "Seeking a New Anti-Skin-Aging Material: Piceatannol and Its Derivatives from Passion Fruit (Passiflora edulis) Seed" In: "ACS Symposium Series", 1 janvier 2013 (2013-01-01), American Chemical Society/Oxford University Press, US, XP055286424, ISSN: 0097-6156 vol. 1129, pages 189-202, DOI: 10.1021/bk-2013-1129.ch012, abstract; page 189 page 191, alinéa 3 page 194, alinéa dernier - page 195, alinéa 1; figure 1 page 196; figure 2 page 197, alinéa dernier - page 198, alinéa 1 page 201, alinéa "conclusion"
- HIROKO MARUKI-UCHIDA ET AL: "The Protective Effects of Piceatannol from Passion Fruit (Passiflora edulis) Seeds in UVB-Irradiated Keratinocytes", BIOLOGICAL & PHARMACEUTICAL BULLETIN (OF JAPAN), vol. 36, no. 5, 1 janvier 2013 (2013-01-01), pages 845-849, XP055286427, JP ISSN: 0918-6158, DOI: 10.1248/bpb.b12-00708
- YUKO MATSUI ET AL: "Extract of Passion Fruit (Passiflora edulis) Seed Containing High Amounts of Piceatannol Inhibits Melanogenesis and Promotes Collagen Synthesis", JOURNAL OF AGRICULTURAL AND FOOD CHEMISTRY, vol. 58, no. 20, 27 octobre 2010 (2010-10-27), pages 11112-11118, XP055030680, ISSN: 0021-8561, DOI: 10.1021/jf102650d

## Description

L'invention se rapporte à un extrait de graines de Passiflore, *Passiflora incarnata* ou *edulis* et préférentiellement *edulis,* particulièrement riche en polyphénols et un procédé de préparation d'un tel extrait. La présente invention porte également sur l'utilisation cosmétique, dermatologique ou thérapeutique d'une telle composition ou d'un tel extrait.

### LES PASSIFLORES

La famille des passiflores (Passiflora) est constituée d'environ 500 espèces. Les espèces sont souvent distribuées dans les régions chaudes tempérées et tropicales et notamment sur le continent américain, mais sont plutôt rares en Asie, Australie et Afrique tropicale.

### Botanique

Les plantes se présentent sont forme d'arbrisseaux ou herbes grimpantes. Les feuilles sont alternes, parfois simples, lobées ou palmées. Les fleurs peuvent attendre 9 cm de diamètre, sont bisexuelles ou unisexuelles et régulières. Elles sont blanches et violettes et présentent des appendices pétaloïdes fins, garnis d'appendices filiformes symbolisant la couronne d'épines du Christ. Le fruit de 4 à 5 cm de longueur est ovalaire et souvent de couleur jaune à orangée.

Les espèces les plus répandues sont notamment *Passiflora incarnata (P. incarnata) et Passiflora edulis (P. edulis).*

### Eléments de Phytochimie

*P. incarnata* : les constituants majeurs sont représentés par la famille des flavonoïdes qui sont présentes en grande quantité dans les feuilles. Les feuilles contiennent, en particulier, une forte teneur en isovitexine. Les feuilles de *P. incarnata* contiennent également en faible quantité des alcaloïdes indoliques simples (harmane, harmine...), des sucres comme le raffinose, le saccharose, fructose, glucose, et des huiles essentielles et du maltol décrit comme la molécule qui serait à l'origine des effets sédatifs et anti-convulsifiants attribués à cette plante.

*P. edulis* : à partir d'un extrait méthanolique de feuilles séchées, un composés spécifiques a été identifié : la passiflorine - cyclopropane triterpène glycoside (E. Bombardelli et al., 1975).

Les feuilles de *P. edulis* contiennent en particulier de l'isoorientine, un flavonoïde qui n'est pas retrouvé dans l'espèce *P. incarnata.* Elles contiennent également des traces d'huile essentielle et d'alcaloïdes identiques à l'espèce *P. incarnata.*

La pulpe du fruit contient des flavonoïdes, schaftoside, isoschaftoside, isoorientine, orientine, isovitexine, dérivés de lutéoline (M.L. Zeraik, J.H. Yariwake - 2010), de l'acide ascorbique (environ 60 mg/100 g).

La pulpe contient également des dérivés cyanogéniques glycosylés : la prunasine, la sambunigrine et l'amygdaline, ainsi que deux mandelonitrile β-rutinosides récemment identifiés (D. Chassagne and J. Crouzet, 1998 ; D.S. Seigler, 2002).

### Toxicologie

Des constituants cyanogéniques sont présents essentiellement dans les parties aériennes de différentes variétés de passiflore.

### Caractéristiques de la graine

Les graines constituent 6 à 12% du fruit de *P. Edulis* et elles contiennent :
- *des polyphénols* dont le Piceatannol (structure proche du resveratrol) et son dimère la scirpusine B (S. Sano ; K. Sugiyama ; T. Ito, 2011), substances à effets vasorelaxant et anti-oxydant.
- *de l'huile,* (rendement de 18% après extraction par solvant) contenant des phytostérols (0.2% dont campestérol, stigmastérol, sitostérol, avenasterol) ; 60 à 73% d'acide linoléique (oméga 6), 14% à 20% d'acide oléique et 465 ppm de tocophérols (G. Piobom, N. Barou et al, 2006 ; R. de V.V. Lopes et al.).
- *Des sucres et des protéines.*

### ART ANTERIEUR

### Usage Alimentaire

Le fruit semble être consommé depuis la préhistoire. Au XVIème siècle au Pérou, les magnifiques fleurs de passiflores étaient déjà considérées comme un remède et de nombreuses espèces de passiflores sont toujours utilisées dans de nombreux pays dans les pratiques thérapeutiques courantes.

### Usage Médical

Les passiflores (souvent les parties aériennes et parfois les fruits) sont souvent utilisées partout dans le monde comme anxiolytique, sédatif, diurétique ou analgésique (« Passiflora : review update. K. Dhawan, S. Dhawan, A. Sharma, 2004 »). Le maltol et certains de ses dérivés seraient à l'origine de cet effet sédatif.

Cette activité serait plus constante et plus significative pour l'espèce *P. incarnata.*

Des extraits de *P. incarnata* seraient capables de lutter contre la dépendance à la morphine.

Un effet anti-hypotenseur d'un extrait méthanolique d'écorces de fruits de *P. edulis,* ainsi qu'un effet hypocholestémiant d'un extrait de graines délipidées riches en fibres ont également été mis en évidence.

Un effet anti-tumoral d'une décoction des fruits via l'inhibition de métalloprotéinases matricielles (MMP2 et MMP9) impliquées dans l'invasion tumorale, les métastases et l'angiogénèse, a également été mis en évidence (S.S. Patel, 2009).

### Usages Dermo-cosmétiques

Au Brésil, les feuilles de *P. foetida* sont utilisées par voie topique pour traiter les maladies cutanées d'origine inflammatoire, en particulier grâce à la présence de l'isoorientine. A Maurice et Rodrigues, les décoctions de feuilles de *P. suberosa* sont également utilisées en bain pour traiter les maladies de peau.

Dans l'article intitulé « Antioxidant activities and phenolics of Passiflora edulis seed recovered from juice production residue" de Lourith et al. (Journal of Oleo Science 2013, 62(4), 235-240), un procédé d'extraction de graines de *Passiflora edulis* est décrit ainsi que les applications notamment cosmétiques de l'extrait obtenu.

Les articles de Matsui et al. intitulés « Seeking a new anti-skin-aging material: piceatannol and its dérivatives from passion fruit (Passiflora edulis) seed" (ACS Symposium Series. 2013, vol.1129, 189-202) et *«* Extract of passion fruit (Passiflora edulis) seed containing high amounts of piceatannol inhibits melanogenesis and promotes collagen synthesis" (J. Agric. Food Chem. 2010, 58, 11112-11118) portent sur les polyphénols contenus dans *Passiflora edulis,* notamment dans les graines de *Passiflora edulis.*

L'article de Maruki-Uchida et al. intitulé « The protective effects of piceatannol from passion fruit (Passiflora edulis) seeds in UVB-irradiated kératinocytes" (Bio. Pharm. Bull. 2013, 36(5), 845-849) décrit des extraits de graines de *Passiflora edulis* contenant du picéatannol et évalue leur activité biologique.

La demande de brevet FR 3 010 906 décrit un extrait lipidique de graines de Passiflore. La demande de brevet KR 2015 0064314 décrit des compositions cosmétiques anti-oxydantes et anti-âges comprenant comme ingrédient actif un extrait végétal, qui peut être un extrait de *Passiflora edulis.*

### DESCRIPTION DE L'INVENTION

La Demanderesse a découvert que les extraits de graines de passiflore, en particulier *Passiflora incarnata* ou *Passiflora edulis,* et avantageusement *Passiflora edulis,* présentent des propriétés cosmétiques, pharmaceutiques et dermatologiques jamais décrites jusqu'à présent. En particulier, c'est la première fois que de tels extraits de graines de passiflore sont utilisés en tant que tels, pour leurs propriétés spécifiques.

L'invention a ainsi pour objet un extrait polyphénolique de graines de Passiflore, en particulier de graines de *Passiflora incarnata* ou de *Passiflora edulis,* plus particulièrement de *Passiflora edulis,* comprenant au moins 30% en poids de polyphénols, exprimés en équivalent d'acide gallique, par rapport au poids de l'extrait sec. Cette teneur équivaut à au moins 3 mg de polyphénols par millilitre d'extrait liquide.

La teneur en polyphénols est exprimée en équivalent d'acide gallique, par rapport au poids de l'extrait sec. Ces pourcentages sont obtenus par un dosage de Folin-Ciocalteu.

Dans un dosage de Folin-Ciocalteu, l'ensemble des composés phénoliques est oxydé par le réactif de Folin-Ciocalteu (disponible dans le commerce). Ce dernier comprend un mélange d'acide phosphotungstique (H3PW12O40) et d'acide phosphomolybdique (H3PMol2O40) qui est réduit, lors de l'oxydation des substances phénoliques, en un mélange d'oxydes bleus de tungstène (W8O23) et de molybdène (Mo8O23). La coloration bleue produite possède une absorption maximum aux environs de 750-760 nm. Elle est proportionnelle à la quantité de composés phénoliques oxydés. Le phénol de référence utilisé dans cette méthode est l'acide gallique (voir par exemple SINGLETON ET AL., Colorimetry of total phenolics with phosphomolybdic-phosphotungstic acid reagents*).* Les résultats obtenus par ce dosage sont donc exprimés en « % en poids de polyphénols, exprimé en équivalent d'acide gallique, par rapport au poids total de l'extrait sec ».

La teneur en polyphénols est donc un paramètre facilement mesurable pour l'homme du métier.

L'extrait selon la présente invention comprend avantageusement au moins 35% en poids de polyphénols, exprimés en équivalent d'acide gallique, par rapport au poids total dudit extrait sec, c'est-à-dire au moins 3,5 mg de polyphénols par millilitre d'extrait liquide.

L'extrait selon la présente invention comprend plus avantageusement au moins 40% en poids de polyphénols, exprimés en équivalent d'acide gallique, par rapport au poids total dudit extrait sec, c'est-à-dire au moins 4 mg de polyphénols par ml d'extrait liquide.

Parmi les polyphénols présents dans l'extrait selon l'invention, on retrouve majoritairement les dérivés catéchiques.

Par « dérivés catéchiques » on entend, au sens de la présente invention, les flavoinoïdes de la famille de la catéchine, également connue sous le nom de catéchol. Les dérivés catéchiques sont plus particulièrement des composés de formule générale (I) suivante : 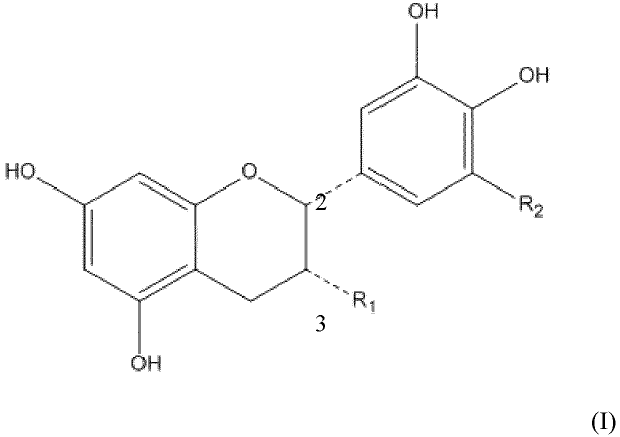 dans laquelle :
------ représente une liaison simple de configuration R ou S ;
R₁ représente OH ou un groupe galloyl de formule (II) suivante : 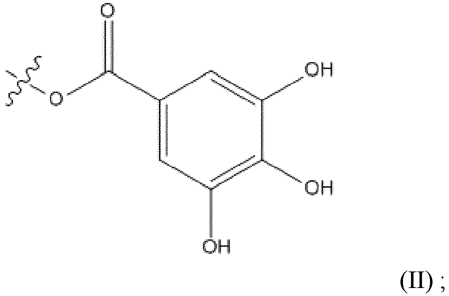 et
R₂ représente H ou OH.

Les dérivés catéchiques sont plus particulièrement des composés de formule générale (I) choisis dans le groupe constitué de :

| Nom | Configuration | **R₁** | **R₂** |
|---|---|---|---|
| (+)-catéchine | 2R, 3S | OH | H |
| (-)-épicatéchine | 2R, 3R | OH | H |
| (-)-catéchine | 2S, 3R | OH | H |
| (+)-épicatéchine | 2S, 3S | OH | H |
| (-)-épigallocatéchine | 2R, 3R | OH | OH |
| (-)-épicatéchine gallate | 2R, 3R | Groupe galloyl | H |
| (-)-épigallocatéchine gallate | 2R, 3R | Groupe galloyl | OH |
| (+)-gallocatéchine | 2R, 3S | OH | OH |
| (+)-gallocatéchine gallate | 2R, 3S | Groupe galloyl | OH |

L'extrait selon l'invention comprend avantageusement au moins 20 % en poids, en particulier au moins 24 % en poids de dérivés catéchiques, exprimés en équivalent d'acide gallique, par rapport au poids de l'extrait sec. Ainsi, dans l'extrait selon l'invention, au moins 50% en poids, en particulier au moins 60% en poids des polyphénols sont des dérivés catéchiques, exprimés en équivalent d'acide gallique, par rapport au poids de polyphénols dans l'extrait sec.

Avantageusement, l'extrait selon l'invention comprend en outre au moins 10% en poids d'acides organiques, notamment d'acide acétique, d'acide malique, d'acide citrique ou leurs mélanges, par rapport au poids de l'extrait sec.

De manière particulièrement avantageuse, l'extrait selon l'invention comprend au moins 30% en poids de polyphénols, exprimés en équivalent d'acide gallique, par rapport au poids de l'extrait sec, et au moins 10% en poids d'acides organiques, notamment d'acide acétique, d'acide malique, d'acide citrique ou leurs mélanges, par rapport au poids de l'extrait sec.

Selon cet aspect, l'extrait selon l'invention a pour avantage d'être riche en acides organiques, ce qui lui confère une forte activité antioxydante, anti-chélatrice et/ou hydratante.

L'extrait selon l'invention est avantageusement obtenu par extraction solide/liquide des graines de Passiflore dans un solvant choisi parmi l'eau, les glycérols, les glycols, et leurs mélanges.

Le solvant est plus particulièrement choisi parmi les mélanges binaires eau/glycérol, eau/glycol, et leurs mélanges, avantageusement dans une proportion comprise entre 30 et 90%, en particulier entre 40 et 90 %, de préférence entre 50 % et 90 %, plus préférentiellement entre 60 % et 80 %, notamment 70 %, de glycérol et/ou de glycol dans l'eau.

De préférence le solvant utilisé est choisi parmi les mélanges binaires eau/glycérol ou eau/propanediol, en particulier eau/propanediol, plus particulièrement eau/1,3-propanediol.

En particulier, l'extrait selon l'invention contient avantageusement en poids par rapport à l'extrait sec obtenu :
- Environ 40 % de polyphénols ;
- Environ 10 % de sucres ;
- Environ 11 % d'acides de fruits ; et
- Environ 5 % de protéines.

De manière particulière, l'extrait selon l'invention ne comprend pas d'isoorientine, orientine, vitexine ou encore d'isovitexine.

L'invention a également pour objet un procédé de préparation d'un extrait polyphénoliques de passiflore en particulier de graines de *Passiflora incarnata* ou de *Passiflora edulis,* avantageusement de *Passiflora edulis,* comprenant au moins 30% en poids de polyphénols, exprimés en équivalent d'acide gallique, par rapport au poids de l'extrait sec, ledit procédé comprenant au moins une étape d'extraction solide/liquide dans un solvant choisi parmi l'eau, les glycérols, les glycols, et leurs mélanges.

Avantageusement, ledit procédé de préparation d'un extrait polyphénolique de graines de passiflore selon l'invention comprend les étapes successives suivantes :
a) broyage des graines ;
b) éventuellement délipidation des graines, de préférence par pressage, extraction éthanolique ou extraction CO2 ;
c) extraction solide/liquide des graines broyées et éventuellement délipidées dans un solvant choisi parmi l'eau, les glycérols, les glycols, et leurs mélanges ;
d) séparation de la phase solide et de la phase liquide par décantation, et/ou centrifugation et/ou filtrations successives ;
e) éventuellement séchage de l'extrait obtenu à l'étape d).

L'étape a) de broyage des graines peut être réalisée par des méthodes connues de l'homme du métier, notamment à l'aide d'un broyeur à couteaux, d'un broyeur à marteaux, etc.

A l'étape c), la phase d'extraction solide/liquide est réalisée de préférence à une température comprise entre 20°C et 90°C, en particulier entre 30°C et 80 °C, plus particulièrement entre 45 °C et 75°C, typiquement 70°C.

La durée d'extraction est avantageusement comprise entre 30 minutes et 4h, en particulier entre 1h et 3h, avantageusement elle est d'environ 2 heures.

Avantageusement, le solvant d'extraction utilisé à l'étape c) est choisi parmi les mélanges binaires eau/glycérol, eau/glycol, et leurs mélanges, avantageusement dans une proportion comprise entre 30 et 90%, en particulier entre 40 et 90 %, de préférence entre 50 % et 90 %, plus préférentiellement entre 60 % et 80 %, notamment 70 %, de glycérol et/ou de glycol dans l'eau. En particulier, le solvant d'extraction est choisi parmi les mélanges binaires eau/glycérol ou eau/propanediol, en particulier eau/propanediol, plus particulièrement eau/1,3-propanediol.

Dans une variante avantageuse du procédé, préalablement à l'étape c), les graines de passiflore sont délipidées. Avant d'être dispersées, les graines broyées peuvent être délipidées, notamment dans de l'éthanol. L'élimination des lipides permet une meilleure efficacité des étapes d'extraction et de filtrations. Il est également et préférentiellement possible d'utiliser des tourteaux de ces graines, c'est-à-dire le résidu issu de l'extraction préalable de l'huile par solvant, technique de CO₂ supercritique par exemple et préférentiellement par pressage mécanique.

L'étape d) de séparation de la phase solide et de la phase liquide est réalisée par des méthodes connues de l'homme du métier, notamment par décantation, centrifugation et/ou filtrations successives jusqu'à parfaite limpidité et propreté microbiologique.

Avantageusement, l'extrait polyphénolique selon l'invention peut être stabilisé par l'étape de séchage e), par des procédés connus de l'homme de l'art.

L'étape de séchage peut, par exemple, être réalisée en présence d'un support de type, par exemple, maltodextrines ou fibres d'acacia (Fibregum^{®} société CNI). La teneur en support varie typiquement selon un ratio allant de 0% à 80% de support par rapport au pourcentage de matière sèche obtenu dans la forme liquide de l'extrait.

L'extrait est préférentiellement séché par lyophilisation afin d'obtenir une poudre finale. La poudre finale comprend avantageusement 30 à 70% en poids de matière sèche de l'extrait, le complément à 100% étant le support de lyophilisation. Plus avantageusement la poudre finale comprend 50% de matière sèche issue de l'extrait et 50% de support de lyophilisation.

Alternativement, la matière première de départ du procédé selon l'invention peut être un tourteau de graines de passiflore délipidées, notamment par pressage. Dans ce cadre et à titre d'exemple non limitatif, l'extrait polyphénolique selon l'invention peut être obtenu selon le procédé suivant :
a') mise en solution de tourteau de graines de passiflore délipidées par pressage, à 10% de matière sèche dans l'eau ;
b') extraction solide/liquide sous agitation pendant 2 heures à une température de 70°C ;
c') purification par étapes de filtrations successives ; et
d') filtration stérile.

L'extrait obtenu par le procédé selon l'invention, tel que décrit dans les paragraphes précédents, comprend avantageusement au moins 35% en poids, plus avantageusement au moins 40% en poids, de polyphénols, exprimés en équivalent d'acide gallique, par rapport au poids de l'extrait sec.

Avantageusement, l'extrait obtenu par le procédé selon l'invention comprend également au moins 10% en poids d'acides organiques, notamment d'acide acétique, d'acide malique, d'acide citrique ou leurs mélanges, par rapport au poids de l'extrait sec.

Avantageusement, l'extrait obtenu par le procédé selon l'invention comprend au moins 20 % en poids, en particulier au moins 24 % en poids de dérivés catéchiques, exprimés en équivalent d'acide gallique, par rapport au poids de l'extrait sec. Ainsi, dans l'extrait obtenu par le procédé selon l'invention, au moins 50% en poids, en particulier au moins 60% en poids des polyphénols sont des dérivés catéchiques exprimés en équivalent d'acide gallique, par rapport au poids de polyphénols dans l'extrait sec.

La présente invention a donc également pour objet un extrait de graines de passiflore en particulier de graines de *Passiflora incarnata* ou de *Passiflora edulis,* avantageusement de *Passiflora edulis,* susceptible d'être obtenu par le procédé mentionné ci-dessus. Un tel extrait répond aux spécifications définies précédemment concernant l'extrait selon l'invention.

Dans la suite de la description, on parlera d'« extrait selon l'invention » pour désigner l'extrait en tant que tel, tel que défini ci-dessus, ou l'extrait susceptible d'être obtenu par le procédé selon l'invention tel que décrit ci-dessus.

L'invention a en outre pour objet une composition comprenant un extrait riche en polyphénols de graines de passiflore selon l'invention, en tant que principe actif, et le cas échéant un excipient approprié. L'extrait selon l'invention est tel que défini dans les paragraphes ci-dessus concernant l'extrait en tant que tel et ceux concernant l'extrait susceptible d'être obtenu par le procédé selon l'invention.

La composition selon l'invention comprend avantageusement de 0,001 à 10% en poids, avantageusement de 0,01 à 5% en poids, dudit extrait polyphénolique de graines de Passiflore selon l'invention, le poids de l'extrait étant exprimé en extrait sec, par rapport au poids total de la composition.

La composition est avantageusement cosmétique, pharmaceutique ou dermatologique. Ladite composition est de préférence formulée pour être administrée par voie topique externe.

La composition selon l'invention peut comprendre en outre un ou plusieurs autres principes actifs.

La composition selon l'invention peut être formulée sous la forme de différentes préparations adaptées à une administration topique.

En particulier, les compositions topiques peuvent être notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Selon sa nature (cosmétique, dermatologique ou pharmaceutique), la composition selon l'invention peut en outre comprendre au moins un excipient cosmétiquement, pharmaceutiquement ou dermatologiquement acceptable. Notamment, la composition selon la présente invention peut en outre comprendre au moins un adjuvant cosmétiquement, pharmaceutiquement ou dermatologiquement connu de l'homme du métier, choisi parmi les tensioactifs, les épaississants, les conservateurs, les parfums, les colorants, des filtres chimiques ou minéraux, les agents hydratants, les eaux thermales, etc. L'homme du métier sait adapter la formulation de la composition selon l'invention en utilisant ses connaissances générales.

Les modes d'administration, les posologies et les formes galéniques optimales des compositions selon l'invention peuvent être déterminés selon les critères généralement pris en compte dans l'établissement d'un traitement pharmaceutique, dermatologique ou cosmétique adapté à un patient ou à un animal, comme par exemple l'âge ou le poids corporel du patient ou de l'animal, la gravité de son état général, la tolérance au traitement, les effets secondaires constatés, le type de peau.

L'invention a également pour objet un extrait selon l'invention ou une composition selon l'invention pour son utilisation pour prévenir et/ou traiter des troubles ou pathologies de la peau et/ou des muqueuses et/ou des phanères, avantageusement des réactions inflammatoires, des réactions d'oxydation, des désordres liés à des attaques radicalaires liées ou non à la pollution, des troubles de la barrière ou de l'homéostasie, du vieillissement, notamment du vieillissement chronologique et/ou actinique, de la peau et/ou des muqueuses et/ou des phanères.

L'invention a également pour objet un extrait selon l'invention ou une composition selon l'invention pour son utilisation pour prévenir et/ou traiter des troubles vasculaires et/ou des altérations du tissu adipeux.

L'invention a également pour objet l'utilisation d'un extrait selon l'invention ou d'une composition selon l'invention pour prévenir et/ou traiter le vieillissement, notamment le vieillissement chronologique et/ou actinique, de la peau et/ou des muqueuses et/ou des phanères.

L'invention a également pour objet l'utilisation d'un extrait de graines de passiflores selon l'invention ou d'une composition selon l'invention, pour la fabrication d'une composition cosmétique, pharmaceutique ou dermatologique pour prévenir et/ou traiter des troubles ou pathologies de la peau et/ou des muqueuses et/ou des phanères, avantageusement des réactions inflammatoires, des réactions d'oxydation, des désordres liés à des attaques radicalaires liées ou non à la pollution, des troubles de la barrière ou de l'homéostasie, du vieillissement, notamment du vieillissement chronologique et/ou actinique, de la peau et/ou des muqueuses et/ou des phanères.

L'invention a également pour objet l'utilisation d'un extrait de graines de passiflores selon l'invention ou d'une composition selon l'invention, pour la fabrication d'une composition cosmétique, pharmaceutique ou dermatologique pour prévenir et/ou traiter des troubles vasculaires et/ou des altérations du tissu adipeux.

La description concerne en outre une méthode pour prévenir et/ou traiter des troubles ou pathologies de la peau et/ou des muqueuses et/ou des phanères, avantageusement des réactions inflammatoires, des réactions d'oxydation, des désordres liés à des attaques radicalaires liées ou non à la pollution, des troubles de la barrière ou de l'homéostasie, du vieillissement, notamment du vieillissement chronologique et/ou actinique, de la peau et/ou des muqueuses et/ou des phanères, comprenant l'administration, notamment l'administration topique, d'une quantité efficace d'un extrait de graines de passiflores selon l'invention ou d'une composition selon l'invention, à un sujet en ayant besoin.

La description concerne en outre une méthode pour prévenir et/ou traiter des troubles vasculaires et/ou des altérations du tissu adipeux, comprenant l'administration, notamment l'administration topique, d'une quantité efficace d'un extrait de graines de passiflores selon l'invention ou d'une composition selon l'invention, à un sujet en ayant besoin.

En particulier, la composition ou l'extrait selon l'invention est destiné(e) à la prévention et/ou au traitement des réactions inflammatoires, des réactions d'oxydation, des désordres liés à des attaques radicalaires liées au stress environnemental, tel que la pollution, les UV, la cigarette, etc., des troubles de la barrière ou de l'homéostasie, du vieillissement, notamment du vieillissement chronologique et/ou actinique, de la peau, des phanères (cheveux et ongles) et/ou des muqueuses (gencives, parodontes, muqueuses génitales) immature(s), normale(s) ou mature(s)/âgée(s).

Notamment, la composition ou l'extrait selon l'invention est destiné(e) à la prévention et/ou au traitement des troubles liés à des réactions inflammatoires et/ou radicalaires provoqués par des expositions UV, et/ou à des polluants, tels que des métaux lourds, ou des réactions intrinsèques, et engendrant ainsi un vieillissement accéléré, des troubles de la barrière, des troubles vasculaires, des rougeurs, etc.

Notamment, la composition ou l'extrait selon l'invention est destiné(e) à lutter contre le vieillissement de la peau, notamment contre le vieillissement chronologique et/ou actinique.

En particulier, l'extrait selon l'invention est destiné à être utilisé en tant qu'agent cosmétique anti-pollution.

On entend par agent cosmétique anti-pollution, un agent qui protège la peau et les matières kératiniques de façon à prévenir, atténuer et/ou supprimer les troubles ou pathologies générées par les gaz toxiques tels que l'ozone et des composés organiques résidus de combustion. Un agent cosmétique anti-pollution a en particulier une activité antioxydante et antiradicalaire. La pollution visée ici est en particulier la pollution atmosphérique (telle que l'ozone), les polluants d'extérieurs (par exemple les dioxydes d'azote, le monoxyde de carbone, le dioxyde de soufre, l'ammoniac, les composés organiques volatiles tels que les hydrocarbures aromatiques polycycliques (par exemple le benzo-α-pyrène)), les polluants d'intérieurs (par exemple les composés organiques volatiles, les résidus de peintures, les biocontaminants, la fumée du tabac, la fumée de cuisson, les matériaux de constructions, les produits d'entretien domestique ou de traitement du bois).

Les troubles ou pathologies de la peau cité(e)s ci-dessus sont plus particulièrement des troubles vasculaires, la dermatite atopique, l'eczéma, la dermatite irritative, la peau sensible, la peau réactive, la peau avec rougeur, l'érythème cutané, la peau âgée ou photoâgée, la peau photosensibilisée, les coups de soleil et les inflammations dues aux rayons de toutes sortes.

La description concerne également un procédé de soin cosmétique de la peau et/ou des phanères et/ou des muqueuses, en vue d'améliorer leur état et/ou leur aspect, consistant à administrer une composition ou un extrait selon la présente invention, avantageusement par voie topique externe.

L'invention concerne un procédé de soin cosmétique de la peau, en vue d'en prévenir le vieillissement, consistant à appliquer sur la peau une composition ou un extrait selon la présente invention.

L'invention concerne également un procédé de traitement cosmétique destiné à obtenir une protection de l'organisme contre les effets de la pollution, consistant à appliquer sur la peau et les phanères, un extrait ou une composition selon l'invention, notamment dans une quantité cosmétiquement efficace.

Les exemples qui suivent permettent d'illustrer l'invention.

### DESCRIPTION DES FIGURES

La Figure 1 représente le pourcentage d'activation du NrF2 par un extrait selon l'invention.
La Figure 2 représente l'Immunomarquage de la Claudine 4 dans des explants de peau traités par le BaP (Protocole 1)
La Figure 3 représente l'Immunomarquage de la filaggrine dans des explants de peau traités par le BaP+Nicotine (Protocole 2)
La Figure 4 représente l'Immunomarquage de la loricrine dans des explants de peau traités par le BaP+Nicotine (Protocole 2)
La Figure 5 représente l'Immunomarquage du Collagène I dans des explants de peau traités par le BaP+Nicotine (Protocole 2)
La Figure 6 représente l'Immunomarquage de l'Elastine dans des explants de peau traités par le BaP (Protocole 1)
La Figure 7 représente l'Immunomarquage de la fibronectine dans des explants de peau traités par le BaP (Protocole 1)
La Figure 8 représente l'aspect visuel de comète après électrophorèse de kératinocytes humains normaux traités. La Figure 8A correspond à un échantillon de contrôle négatif, la Figure 8B à un échantillon de contrôle UV et la Figure 8C à un extrait selon l'invention à 0.001 %.
La Figure 9 représente l'évolution des teneurs en métaux lourds à T0 et à T28 chez les sujets étudiés.
La Figure 10 représente l'évolution des teneurs MDA (Figure 10A), Catalase et SOD (Figure 10B), exprimées en U/mg de protéines, à T0 et à T28 chez les sujets ayant reçus le placebo ou ayant reçus l'actif.
La Figure 11 représente l'évolution de la teneur en protéines carbonylées à T0 et à T28 chez les sujets ayant reçus le placebo ou ayant reçus l'actif. La Figure 11A est sous forme d'un diagramme à barre et la Figure 11B représente des photographies de couches cellulaires avec marquage fluorescent des protéines carbonylées. La quantification est effectuée ensuite par traitement d'image.

### EXEMPLE

### Exemple 1 : extrait selon l'invention

Un extrait polyphénolique est obtenu selon le procédé suivant :
a) mise en solution de tourteau de graines de *Passiflore edulis* délipidées par pressage et broyées, à 10% de matière sèche dans un mélange 1, 3-propanediol / eau 70/30 p/p ;
b) extraction sous agitation 2h à 70°C ;
c) élimination de la plante résiduelle par filtration grossière ; et
d) purification de l'extrait obtenu par filtrations dont une filtration stérile supplémentaire.

L'extrait polyphénolique liquide ainsi obtenu présente les caractéristiques suivantes (% sur Extrait Sec):
- Extrait sec (étuve): 0,9% (m/m)
- Teneur en polyphénols totaux (par spectrophotométrie - en équivalent acide gallique) : 43%
- Teneur en dérivés catéchiques (par HPLC - en équivalent acide gallique) : 22,3 %
- Teneur en acide malique (par kit enzymatique) : 5,4%
- Teneur en acide citrique (par kit enzymatique): 3,7%
- Teneur en Protéines (par Kjeldahl x 6.25) : 4,5%

### Exemple 2 : extrait selon l'invention

Un extrait polyphénolique est obtenu selon le procédé suivant :
a) mise en solution de 13.3% graines de *Passiflore edulis* non délipidées et broyées dans un mélange 1, 3 - propanediol / Eau 70/30
b) extraction sous agitation 2h à 70°C
c) élimination de la plante résiduelle par filtration grossière
d) purification de l'extrait obtenu par filtrations, dont une filtration stérile supplémentaire.

L'extrait polyphénolique liquide ainsi obtenu présente les caractéristiques suivantes (% sur Extrait Sec) :
- Extrait sec (étuve) : 1.07 % (m/m)
- Teneur en polyphénols totaux (par spectrophotométrie - équivalent acide gallique) : 40%
- Teneur en Protéines (Kjeldahl x 6.25) : 5,7%

### Exemple 3 : Activité biologique de l'extrait selon l'invention

### 1. Potentiel biologique

Le potentiel Biologique d'un extrait selon l'invention a été recherché grâce à un test de modulation de l'expression génique sur des Fibroblastes Humains Normaux (FHN) et sur des épidermes humains reconstruits et mélanisés.

Ainsi, l'expression de 46 gènes impliqués dans divers voies physiologiques de l'Epiderme (Barrière, pigmentation, inflammation...) et du Derme (Cicatrisation, élasticité, fermeté...) a été étudiée par PCR-array.

### Matériel et méthodes :

Des fibroblastes humains normaux (FHN) et des épidermes reconstruits mélanisés ont été incubés pendant 24h en présence d'un extrait selon l'invention, tel qu'obtenu dans l'exemple 1, à 0,002% et 0,05% (p/v) pour les FHN ou à 0,002% et 0,005% (p/v) pour les épidermes reconstruits, et en présence de TGF-β1 à 20ng/ml sur les FHN ou en présence de Vitamine D3 à 1nM sur les épidermes reconstruits (contrôles de validation des essais).

Au terme du traitement, les ARN ont été extraits et les expressions géniques ont été analysées par qRT-PCR à l'aide de carte TaqMan ciblant les fonctions clés du derme et de l'épiderme.

### Résultats et conclusion:

Les résultats sont présentés dans les tableaux 2 et 3 ci-dessous et montrent notamment que l'extrait selon l'invention, en faisant varier l'expression génique de certains marqueurs, a un intérêt particulier dans les activités suivantes :
- l'homéostasie et la structure de la matrice extracellulaire dermique (**↘** MMP3) ; et
- la Jonction Dermo-Epidermique (71 LAMC2).
Plus particulièrement, les polyphénols de l'extrait selon l'invention ont permis de moduler l'expression génique de gènes impliqués dans les défenses anti-oxydantes et le phénomène de l'Hormesis (71 HMOX1, FTL et G6PD).

Cela démontre que l'extrait selon l'invention a une activité antioxydante, anti-radicalaire et anti-âge.

**Tableau 2 - Screening sur FHN**

| | **Extrait selon l'invention à 0,002%** | | **Extrait selon l'invention à 0,05%** | |
|---|---|---|---|---|
| | QR^{∗} | p-value | QR^{∗} | p-value |
| Heme oxygenase (HMOX1) | - | - | 4,0641 | 0,0025 |
| Ferritin, Light polypeptide (FTL) | - | - | 2,3263 | 0,0221 |
| Glucose-6-phosphate dehydrogenase (G6PD) | - | - | 1,7197 | 0,0218 |
| Matrix metalloproteinase 1 (MMP1) | - | - | 0,6052 | 0,0282 |
| Matrix metalloproteinase 3 (MMP3) | 0,5513 | 0,0173 | - | - |

| | | | | |
|---|---|---|---|---|
| ^{∗} *Niveau d'expression génique exprimé en quantité relative (QR) par rapport au contrôle non traité =1* | | | | |

**Tableau 3 - Screening sur Epidermes reconstruits**

| | **Extrait selon l'invention à 0,002%** | | **Extrait selon l'invention à 0,05%** | |
|---|---|---|---|---|
| | QR^{∗} | p-value | QR^{∗} | p-value |
| Laminin subunit gamma-2 (LAMC2) | 2,2309 | 0,0387 | 4,1885 | 0,0165 |
| Proopiomelanocortin (POMC) | 2,3447 | 0,0398 | 1,7019 | 0,0183 |
| Melanocyte-stimulating hormone receptor (MC1R) | 0,7109 | 0,0175 | 0,6188 | 0,0127 |
| Cytosolic phospholipase A2 (PLA2G4A) | 0,6829 | 0,0391 | - | - |
| L-dopachrome tautomerase (DCT) | - | - | 0,4637 | 0,0296 |

| | | | | |
|---|---|---|---|---|
| ^{∗} *Niveau d'expression génique exprimé en quantité relative (QR) par rapport au contrôle non traité =1* | | | | |

### 2. Activité inductrice de l'Hormesis et de la détoxification cellulaire

Une molécule Hormétique (ou Hormétine) est une substance ayant un effet biphasique, qui à faible dose va avoir un effet bénéfique mais qui à forte dose aura l'effet inverse (Ex : Prooxydant ou Antioxydant). Une Hormétine est également décrite comme étant une molécule reproduisant les effets d'un léger stress sur l'organisme mais qui en retour va permettre à la cellule de se protéger de futures agressions et ainsi protéger l'organisme de diverses maladies (cancers) ou phénomènes physiques liés à l'âge (vieillissement cutanée, mauvaise cicatrisation...) ou encore des effets nocifs de l'environnement (UV, pollution...).

Les analyses suivantes permettent d'étudier l'activité inductrice de l'Hormesis et de la détoxification cellulaire d'un extrait selon l'invention.

### A- Activation de la translocation du facteur de transcription NrF2 :

L'effet de l'extrait selon l'invention a été évalué vis-à-vis de l'activation de la translocation du Nrf2, précurseur de la cascade responsable de la réponse hormétique et de certaines voies de détoxification de l'organisme.

### Matériel et méthodes

Des kératinocytes HaCaT transfectés ARE-Luciférase, contenant le plasmide Nqo1 Antioxydant Response Element (ARE) qui est un plasmide spécifique de l'activation du NrF2 et la luciférase (gène rapporteur), ont été traités pendant 6h à 37°C par un extrait selon l'invention, tel qu'obtenu dans l'exemple 1, à des concentrations allant de 0,01% à 0,0005% (p/v) et par une référence positive le Ter-butylhydroquinone à 20µM.

A la fin du traitement les tapis cellulaires sont lysés, l'activité de la luciférase est dosée grâce à un « Luciférase Assay Kit » de chez PROMEGA et la teneur en protéines de chaque lysat est dosée par la méthode de Bradford (BioRad).

### Résultats et conclusion

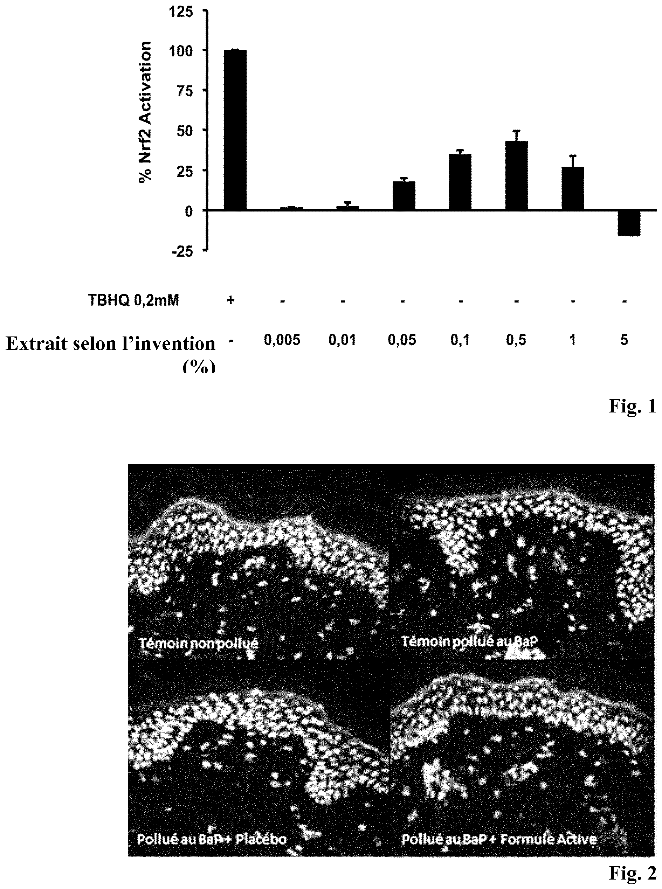

Les résultats obtenus sont présentés dans le tableau 4 et à la Figure 1. Ces résultats montrent que l'extrait selon l'invention a induit une augmentation de l'activité du Nrf2 dans les noyaux et donc une translocation de celui-ci à travers la membrane nucléaire montrant une activation de ce facteur de transcription.

Cela démontre que l'extrait selon l'invention a une activité antioxydante, anti-radicalaire et anti-âge.

**Tableau 4 - Activité Nrf2 dans des HaCaT ARE-Luciférase (RLU = Relative Luciférase Activity)**

| | **Activité NRF2 (moyenne normalisée RLU/µg de protéines)** | **Activation (%)** | |
|---|---|---|---|
| Control | 0 | 0 | |
| Tert-butylhydroquinone 20µM | 23216,4 | 100 | ^{∗∗∗} |
| Extrait selon l'invention à 0,0005% | 4149,6 | 18 | ^{∗} |
| Extrait selon l'invention à 0,001% | 8178,2 | 35 | ^{∗∗∗} |
| Extrait selon l'invention à 0,005% | 9998,6 | 43 | ^{∗∗∗} |
| Extrait selon l'invention à 0,01% | 6324,6 | 27 | ^{∗∗∗} |

| | | | |
|---|---|---|---|
| ^{∗} *0,01<p<0,05;* ^{∗∗} *0,001<p<0,01;* ^{∗∗∗}*p<0,001 et ns = non significatif vs cellules contrôles ANOVA à un facteur suivie d'un test de Dunnett* | | | |

### B- Expression génique des marqueurs principaux de l'Hormesis et de la détoxification cellulaire.

L'effet d'un extrait selon l'invention a été étudié sur l'expression génique de différents marqueurs impliqués dans les voies de l'Hormesis et dans la détoxification cellulaire.

### Matériel et méthodes :

Des Fibroblastes humains normaux, ont été traités pendant 6h, 24h et 48 heures à 37°C par un extrait selon l'invention, tel qu'obtenu dans l'exemple 1, à 0,005% et 0,002% (p/v).

A la fin du traitement, l'expression génique des marqueurs de l'Hormesis (HMOX1, FTL, G6PD et Nrf2) ainsi que des marqueurs impliqués dans la détoxification cellulaire (la SOD1 et la Catalase) a été analysée par RT-PCR quantitative en temps réel et normalisée vis-à-vis du gène de ménage HPRT (technologie SybrGreen).

Les résultats ont été analysés statistiquement par une ANOVA à un facteur suivie d'un test de Dunnett (Logiciel GraphPad Prism version 5.02, GraphPad Software, San Diego California USA).

### Résultats :

Les résultats obtenus sont présentés dans le tableau 5. Ces résultats montrent que l'extrait selon l'invention a significativement stimulé l'expression génique de la HMOX1 en 6h et 24h, de FTL aux trois temps, de la G6PD en 6h et 24h, de la SOD1 en 24h, du NrF2 en 24h et 48h ainsi que la Catalase en 24h.

Cela démontre que l'extrait selon l'invention a une activité antioxydante, anti-radicalaire et anti-âge.

**Tableau 5 - Expression génique des marqueurs de l'Hormesis dans des fibroblastes humains normaux (Quantité Relative)**

| 6h | | HMOX1 | | | | FTL | | G6PD | | SOD1 | Nrf2 | Catalase |
|---|---|---|---|---|---|---|---|---|---|---|---|---|
| Cellules contrôles | | 1,00 | | | | 1,00 | | 1,00 | | 1,00 | 1,00 | 1,00 |
| Extrait selon l'invention à 0,002% | | 3,38 (+238% ***) | | | | 0,94 (-6% ns) | | 1.31 (+31% *) | | 1.03 (+3% ns) | 1.00 (+0% ns) | 0.97 (-3% ns) |
| Extrait selon l'invention à 0,005% | | 23.06 (+2206% ***) | | | | 1.37 (+37% ***) | | 1.31 (+31%*) | | 1.05 (+5% ns) | 1.04 (+4% ns) | 1.02 (+2% ns) |
| | | | | | | | | | | | | |

| 24h | HMOX1 | | | FTL | | | G6PD | | SOD1 | | Nrf2 | Catalase |
|---|---|---|---|---|---|---|---|---|---|---|---|---|
| Cellules contrôles | 1,00 | | | 1,00 | | | 1,00 | | 1,00 | | 1,00 | 1,00 |
| Extrait selon l'invention à 0,002% | 1.66 (+66% ***) | | | 1.62 (+62% **) | | | 1.41 (+41% ns) | | 1.13 (+13% ns) | | 1.25 (+25% *) | 1.23 (+23% ns) |
| Extrait selon l'invention à 0,005% | 2.68 (+168% ***) | | | 2.40 (+140% ***) | | | 1.70 (+70% **) | | 1.33 (+33% *) | | 1.30 (+30% *) | 1.31 (+31% *) |
| | | | | | | | | | | | | |

| 48h | | | HMOX1 | | FTL | | G6PD | | SOD1 | | Nrf2 | Catalase |
|---|---|---|---|---|---|---|---|---|---|---|---|---|
| Cellules contrôles | | | 1,00 | | 1,00 | | 1,00 | | 1,00 | | 1,00 | 1,00 |
| Extrait selon l'invention à 0,002% | | | 0.98 (-2% ns) | | 1.10 (+10% ns) | | 0.88 (-12% ns) | | 1.39 (+39% ns) | | 1.34 (+34%*) | 1.06 (+6% ns) |
| Extrait selon l'invention à 0,005% | | | 1.00 (0% ns) | | 1.66 (+66% ***) | | 0.86 (-14% ns) | | 0.97 (-3% ns) | | 1.26 (+26% ns) | 1.04 (+4% ns) |

| | | | | | | | | | | | | |
|---|---|---|---|---|---|---|---|---|---|---|---|---|
| ^{∗} *0,01<p<0,05;* ^{∗∗} *0,001<p<0,01;* ^{∗∗∗} *p<0,001 et ns* = *non significatif vs cellules contrôles ANOVA à un facteur suivie d'un test de Dunnett* | | | | | | | | | | | | |

### C- Production de la Heme Oxygenase :

Un extrait selon l'invention a été analysé sur l'expression protéique de la Heme Oxygénase.

### Matériel et méthodes :

Des fibroblastes humains normaux, ont été traités pendant 24h, par un extrait selon l'invention, tel qu'obtenu dans l'exemple 1, à 0,002% et 0,005% (p/v) et par le Curcumin à 5 et 10µM (Hormétine de référence).

A la fin du traitement la Hème Oxygénase 1 (ou HMOX1 ou HO1) intracellulaire a été quantifiée par une technique ELISA. La coloration, proportionnelle à la quantité du marqueur étudié, a été mesurée par lecture de la densité optique (DO) à 450nm et la valeur obtenue a été ramenée à la quantité de cellules obtenue par un dosage des protéines par la technique du BC Assay (Interchim).

Les résultats ont été analysés statistiquement par une ANOVA à un facteur suivie d'un test de Dunnett (Logiciel GraphPad Prism version 5.02, GraphPad Software, San Diego California USA).

### Résultats :

Les résultats obtenus sont présentés dans le tableau 6. Ces résultats montrent que l'extrait selon l'invention a une induction de même intensité que le Curcumin sur la production de la Heme Oxygénase 1. Ce résultat confirme l'action de l'extrait selon l'invention que nous avons observée lors de l'étude de l'expression génique de ce même marqueur.

Cela démontre que l'extrait selon l'invention a une activité antioxydante, anti-radicalaire et anti-âge.

**Tableau 6 - Production d'HMOX1 par des fibroblastes**

| | **HMOX1 (ng/ml sur quantité de cellules)** | **Inhibition** |
|---|---|---|
| Cellules contrôles | 0.033 ± 0.003 | |
| Curcumin à 5µM | 0.060 ± 0.002 | 82% ^{∗∗∗} |
| Curcumin à 10µM | 0.105 ± 0.006 | 218% ^{∗∗∗} |
| Extrait selon l'invention à 0,002% | 0.056 ± 0.002 | 69% ^{∗∗∗} |
| Extrait selon l'invention à 0,005% | 0.098 ± 0.004 | 197% ^{∗∗∗} |

| | | |
|---|---|---|
| ^{∗∗∗} *p*<*0,001 vs cellules contrôles* - *ANOVA à un facteur suivie d'un test de Dunnett D- Production de la Heme Oxygenase en condition siRNA Nrf2 :* | | |

Afin de vérifier si la voie d'activation de la hème oxygénase par un extrait selon l'invention passe bien par une activation du Nrf2, le potentiel d'induction de la production de Heme Oxygenase a été vérifié dans un système où l'expression du Nrf2 est bloquée (small interférence siRNA).

### Matériel et méthodes :

Des fibroblastes humains normaux, ont été prétraités pendant 24h par du siRNA Nrf2 et par du siRNA Scrambled (témoin siRNA sans actions) puis, sur chacune des conditions précédentes, pendant 24h par un extrait selon l'invention, tel qu'obtenu dans l'exemple 1, à 0,005% (p/v).

A la fin du traitement la Hème Oxygénase 1 (ou HMOX1) intracellulaire a été quantifiée par une technique ELISA. La coloration, proportionnelle à la quantité du marqueur étudié, a été mesurée par lecture de la densité optique (DO) à 450nm.

Les résultats ont été analysés statistiquement par une ANOVA à un facteur suivie d'un test de Tukey (Logiciel GraphPad Prism version 5.02, GraphPad Software, San Diego California USA).

### Résultats :

Les résultats obtenus sont présentés dans le tableau 7. Ces résultats montrent que la stimulation de la production d'HMOX1 induite par un extrait selon l'invention est largement diminuée (-62%, p<0,001) lorsque la voie Nrf2 est partiellement bloquée (siRNA Nrf2).

L'activité d'un extrait selon l'invention sur la production d'Hème Oxygénase passe donc bien par la voie du Nrf2.

Cela démontre que l'extrait selon l'invention a une activité antioxydante, anti-radicalaire et anti-âge.

**Tableau 7 - Production d'HMOX1 par des fibroblastes - Comparatif siRNA Scrambled vs siRNA Nrf2**

| | HMOX1 (ng/ml) | Inhibition liée au siRNA Nrf2 | |
|---|---|---|---|
| Cellules contrôles Scrambled | 3,371 ± 0.702 | - | - |
| Cellules contrôles siRNA Nrf2 | 2.199 ± 0.089 | -35% | ^{∗} |
| Extrait selon l'invention à 0,005% Scrambled | 18.821 ± 0.316 | - | - |
| Extrait selon l'invention à 0,005% siRNA Nrf2 | 7.105 ± 0.131 | -62% | ^{∗∗∗} |

| | | | |
|---|---|---|---|
| ^{∗∗∗} *p<0, 001 vs cellules contrôles* - *ANOVA à un facteur suivie d'un test de Tukey* | | | |

### E- Effet sur la production d'espèces réactives de l'oxygène (ERO):

Le potentiel antioxydant d'un extrait selon l'invention vis-à-vis de l'induction d'espèces réactives de l'oxygène induit par de l'H₂O₂ a été étudié.

### Matériel et méthodes :

Des kératinocytes humains normaux ont été incubés, pendant 24 heures, en présence d'un extrait selon l'invention, tel qu'obtenu dans l'exemple 1, à 0,002%; 0,005% et 0,01% (p/v) ou de la vitamine C à 500µM et de la Quercétine à 10µM (Antioxydants de références) avant incorporation de la sonde H2DCF-DA (incubation de 60 minutes).

Les kératinocytes ont ensuite été stimulés par du peroxyde d'hydrogène (H₂O₂) à 100 µM pendant 20 minutes et la production d'ERO (Espèces Réactives de l'Oxygène) a été évaluée par mesure de fluorescence.

Les résultats ont été analysés statistiquement par une ANOVA à un facteur suivie d'un test de Tukey (Logiciel GraphPad Prism version 5.02, GraphPad Software, San Diego California USA).

### Résultats :

Les résultats obtenus sont présentés dans le tableau 8. Ces résultats montrent que l'extrait selon l'invention a significativement inhibé la production d'ERO par des kératinocytes en réponse à un stress oxydant induit par le peroxyde d'hydrogène (H2O2). Cette activité antioxydante étant d'un niveau équivalent à celle des deux témoins antioxydants (Vitamine C et Quercétine).

Cela démontre que l'extrait selon l'invention a une activité antioxydante, anti-radicalaire et anti-âge.

**Tableau 8 - Production d'ERO dans des kératinocytes traités par H₂O₂**

| | **ERO (Unités de fluorescence)** | **Significativité** | |
|---|---|---|---|
| Cellules contrôles | 30752.921 ± 4555.136 | - | - |
| Cellules stimulées (H₂O₂) | 44179.976 ± 7445.110 | +44% | ^{∗∗∗} |
| Référence (Vit. C) | 21719.018 ± 3174.253 | -51% | ^{∗∗∗} |
| Référence (Quercétine) | 14367.847 ± 1790.753 | -67% | ^{∗∗∗} |
| Extrait selon l'invention à 0,002% | 18769.676 ± 2780.206 | -58% | ^{∗∗∗} |
| Extrait selon l'invention à 0,005% | 14404.891 ± 2974.938 | -67% | ^{∗∗∗} |
| Extrait selon l'invention à 0,01% | 16531.685 ± 1902.378 | -63% | ^{∗∗∗} |

| | | | |
|---|---|---|---|
| ^{∗∗∗} *p<0,001 - ANOVA à un facteur suivie d'un test de Tukey* | | | |

### 3. Protection contre les effets néfastes de la pollution

Les précédents résultats ont montré qu'un extrait selon l'invention stimule la production de l'hème oxygénase 1 via l'activation de la translocation du facteur de transcription Nrf2. En conséquence, l'extrait selon l'invention a permis une protection cellulaire antioxydante via la diminution de la formation des ERO induits par un stress H2O2.

L'extrait selon l'invention permettant de stimuler les défenses de la peau, nous avons évalué leur effet protecteur vis-à-vis de divers stress environnementaux, ici la pollution.

### A- Effet sur le stress oxydant :

### Matériel et méthodes :

Des kératinocytes humains normaux ont été traités pendant 24 heures par un extrait selon l'invention, tel qu'obtenu dans l'exemple 1, à 0,002% (p/v), la vitamine C à 500µM et la Quercétine à 10µM (Antioxydants de référence), par le Curcumin à 10µM (Hormétine de référence) ou par le Resveratrol à 10µM avant incorporation de la sonde H2DCF-DA (incubation de 45 minutes).

Les kératinocytes ont ensuite été stimulés par du Benzo-α-pyrène (BaP) à 9 µg/ml pendant 20 minutes.

La production d'ERO a été évaluée par mesure de fluorescence.

La significativité des résultats a été vérifiée par un test t de Student.

### Résultats et conclusion :

Les résultats obtenus sont présentés dans le tableau 9. Ces résultats montrent que l'extrait selon l'invention à 0,002% a inhibé la production de ROS par des kératinocytes en réponse à un stress oxydant induit par le BaP à 6 et 9µg/ml.

Donc l'extrait selon l'invention exerce un effet protecteur vis-à-vis du stress oxydant induit par la pollution. L'extrait selon l'invention a donc une activité antioxydante, anti-radicalaire, anti-pollution et anti-âge.

**Tableau 9 - Production d'ERO dans des kératinocytes traités par du BaP à 9 µg/ml**

| | **ROS** (Unités de fluorescence) | **Significativité** | |
|---|---|---|---|
| Cellules contrôles | 4730 ± 324 | - | - |
| Cellules stimulées (BaP à 9µg/ml) | 16777 ± 1755 | +255% | *** |
| Référence (Vit. C) | 14089 ± 1719 | -16% | NS |
| Référence (Quercétine) | 17140 ± 930 | -32% | NS |
| Curcumin à 10µM | 14065 ± 1231 | -16% | NS |
| Resveratrol à 10µM | 20357 ± 503 | -21% | * |
| **Extrait selon l'invention à 0,002%** | **11052 ± 828** | **34%** | ****** |

| | | | |
|---|---|---|---|
| ^{∗} *p*<*0,05;* ^{∗∗}*p*<*0,01 ;* ^{∗∗∗}*p<0,001 et ns* = *non significatif* - *test t de Student* | | | |

### B- Protection des structures cutanées :

La capacité d'un extrait selon l'invention à protéger l'intégrité de la peau (derme et épiderme) des effets néfastes de la pollution a été étudiée sur des explants de peau humaine.

### Matériel et méthodes :

Des explants de peau humaine, provenant d'une femme de 45 ans, ont été prétraités pendant 24h par une application topique d'une formule cosmétique contenant ou non (placébo) un extrait selon l'invention, tel qu'obtenu dans l'exemple 1 à 3%.

Ces explants ont ensuite à nouveau été traités par les formules cosmétiques en présence de Benzo-α-Pyréne (BaP à 20µM) pour le protocole 1 ou de Bap+Nicotine (20µM) pour le protocole 2.

Des Immunomarquages de différents marqueurs des structures cutanées ont été réalisés.

### Résultats et conclusion :

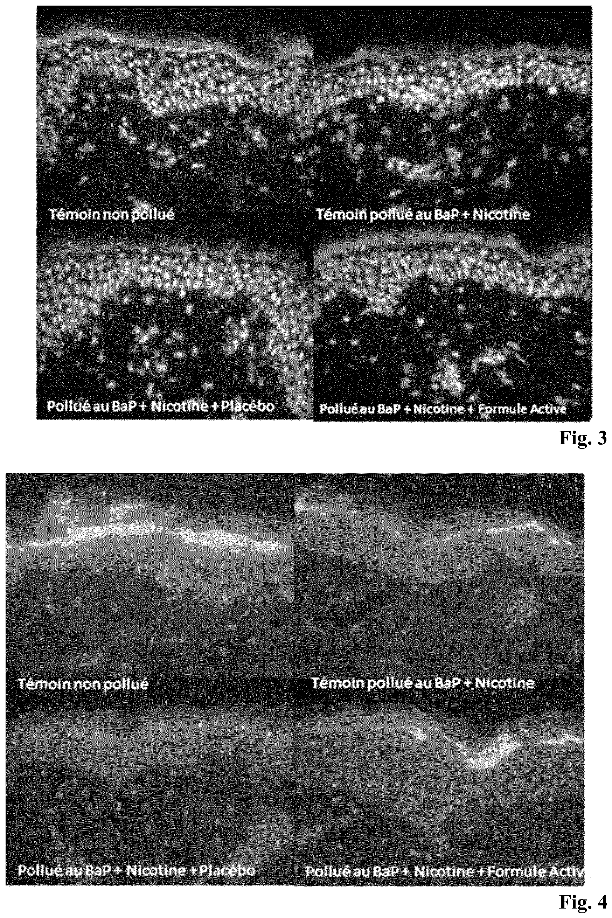

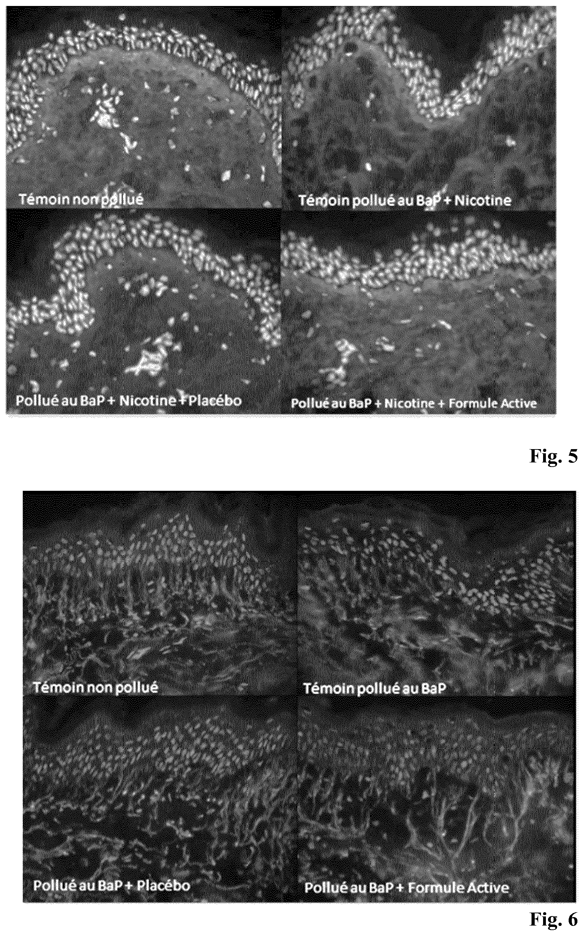

Les résultats obtenus sont présentés par les Figures 2 à 7.

Le stress mimant la pollution induit par le BaP +/- Nicotine a conduit à une altération de l'expression des marqueurs structurels étudiés.

Dans ces conditions, l'extrait selon l'invention a protégé les marqueurs épidermiques suivants :
- la Claudine 4 (Marqueur des jonctions serrées/fonction barrière),
- la Filaggrine (Marqueur de la fonction barrière et précurseur des facteurs naturels d'hydratation), et
- la Loricrine (Marqueur impliqué dans la différenciation cellulaire/fonction barrière) ;

Ainsi que les marqueurs dermiques comme :
- le Collagène I (Marqueur impliqué dans la fermeté de la peau),
- l'Elastine (Marqueur impliqué dans l'élasticité de la peau), et
- de la Fibronectine (Marqueur impliqué dans la structure du Derme).

Ces résultats montrent un réel effet protecteur de l'intégrité structurelle de l'épiderme et de sa fonction barrière ainsi que le maintien d'une structure normale du derme et donc une action protectrice globale de la peau vis-à-vis de la pollution environnementale.

L'extrait selon l'invention a donc une activité antioxydante, anti-radicalaire, anti-pollution et anti-âge.

### C- Evaluation de l'activité détoxifiante vis-à-vis d'un stress à l'ozone

Le potentiel protecteur et détoxifiant d'un extrait selon l'invention vis-à-vis d'un stress à l'ozone a été évalué sur des épidermes reconstruits.

### Matériel et méthodes :

Des épidermes humains reconstruites (RHE) ont été prétraités pendant 24 heures par une application topique d'une formule cosmétique contenant ou non (placébo) un extrait selon l'invention, tel qu'obtenu dans l'exemple 1 à 3%.

Les épidermes ont ensuite été soumis à un stress à l'ozone de 0,5 à 1ppm.

Les activités enzymatiques des enzymes détoxifiantes Catalase, Glutathion Peroxidase (GPX) et SuperOxyde Dismutase (SOD) ont été quantifiées par une méthode ELISA en colorimétrie (SOD et GPX) ou en fluorescence (Catalase).

La peroxydation lipidique a été évaluée par le dosage du MalonDiAldéhyde (MDA), réalisé par CPG.

L'oxydation de l'ADN a été évaluée par le dosage de la 8-HydroxydésoxyGuanosine (8-OHdG), réalisé par colorimétrie.

Les résultats ont été analysés statistiquement par une ANOVA à un facteur suivie d'un test de Tukey (Logiciel GraphPad Prism version 5.02, GraphPad Software, San Diego California USA).

### Résultats et conclusion :

Le stress à l'ozone a induit une suractivation des enzymes Catalase, SOD et GPX ; cette augmentation témoigne du stress oxydant induit par l'ozone.

Dans ces conditions, l'extrait selon l'invention a significativement inhibé cette suractivation.

En absence de stress à l'ozone (condition basale), l'extrait selon l'invention n'induit aucune inhibition de l'activité des enzymes détoxifiantes. Ceci confirme qu'il s'agit bien d'un effet protecteur vis-à-vis du stress induit et non d'une inhibition des systèmes de détoxification de la cellule.

### (Tableaux 10, 11 et 12).

Le stress à l'ozone a induit des dommages oxydatifs qui se traduisent par une augmentation de la 8-OHdG et du MDA.

L'extrait selon l'invention protège significativement les éléments cellulaires des dommages oxydatifs induits par le stress à l'ozone en inhibant significativement la production de 8-OHdg et de MDA.

### (Tableaux 13 et 14).

**Tableau 10 - Activité de la Catalase dans des RHE traités ou non par de l'Ozone**

| **Dosage de la catalase en condition de stress à l'Ozone** | | | |
|---|---|---|---|
| | **Moyenne** | **% d'évolution** | **Tukey** |
| Epidermes Contrôles | 156,1 | 0 | |
| Ozone | 340,1 | 118 | $$$ |
| Placébo | 306,4 | -10 | ns |
| Extrait selon l'invention à 3% | 244,2 | -28 | *** |
| | | | |

| **Dosage de la catalase en condition basale** | | | |
|---|---|---|---|
| | **Moyenne** | **% d'évolution** | **Tukey** |
| Contrôle | 181,8 | 0 | |
| Placébo | 250,3 | 38 | $$$ |
| Extrait selon l'invention à 3% | 231,4 | 27 | $$ |

| | | | |
|---|---|---|---|
| *$$ 0,001<p<0, 01; $$$ p<0,001 vs Contrôle* ^{∗∗} *0,001<p<0,01;* ^{∗∗∗} *p*<*0,001 vs Ozone* | | | |

**Tableau 11 - Activité de la GPX dans des RHE traités ou non par de l'Ozone**

| **Dosage de la GPX en condition de stress à l'ozone** | | | |
|---|---|---|---|
| | **Moyenne** | **% d'évolution** | **Tukey** |
| Contrôle | 13,0 | 0 | |
| Ozone | 34,9 | 168 | $$$ |
| Placébo | 31,1 | -11 | * |
| Extrait selon l'invention à 3% | 23,3 | -33 | *** |
| | | | |

| ***Dosage de la GPX en condition basale*** | | | |
|---|---|---|---|
| | **Moyenne** | **% d'évolution** | **Tukey** |
| Contrôle | 13,0 | 0 | |
| Placébo | 12,3 | -6 | ns |
| Extrait selon l'invention à 3% | 13,2 | 2 | ns |

| | | | |
|---|---|---|---|
| *$$$ p*<*0,001 vs Contrôle-* ^{∗} *0,01*<*p*<*0,05;* ^{∗∗∗} *p*<*0,001 vs Ozone* | | | |

**Tableau 12 - Activité de la SOD dans des RHE traités ou non par de l'Ozone**

| **Dosage de la SOD en condition de stress à l'ozone** | | | |
|---|---|---|---|
| | Moyenne | % d'évolution | Tukey |
| Contrôle | 5,7 | 0 | |
| Ozone | 13,0 | 130 | $$$ |
| Placébo | 9,5 | -27 | *** |
| Extrait selon l'invention à 3% | 7,2 | -45 | *** |
| | | | |

| **Dosage de la SOD en condition basale** | | | |
|---|---|---|---|
| | Moyenne | % d'évolution | Tukey |
| Témoin - | 4,7 | 0 | |
| Formule Placébo | 5,4 | 15 | $$ |
| Extrait selon l'invention à 3% | 4,9 | 4 | ns |

| | | | |
|---|---|---|---|
| *$ 0,01*<*p*<*0,05; $$ 0,001*<*p*<*0,01 $$$ p*<*0,001 vs Contrôle* ^{∗∗} *0,001*<*p*<*0,01;* ^{∗∗∗} *p*<*0,001 vs Ozone* | | | |

**Tableau 13 - Dosage de la 8-oxo-dg dans des RHE traités par de l'Ozone**

| | **Moyenne** | **% d'évolution** | **Tukey** |
|---|---|---|---|
| Contrôle | 2,9 | 0 | |
| Ozone | 7,4 | 155 | $$$ |
| Placébo | 5,4 | -27 | ** |
| Extrait selon l'invention à 3% | 3,7 | -51 | *** |

| | | | |
|---|---|---|---|
| *$$$ p*<*0,001 vs Contrôle* ^{∗} *0,01<p<0,05;* ^{∗∗} *0,001*<*p*<*0,01;* ^{∗∗∗} *p*<*0,001 vs Ozone* | | | |

**Tableau 14 - Dosage de la MDA dans des RHE traités par de l'Ozone**

| | **Moyenne** | **% d'évolution** | **Tukey** |
|---|---|---|---|
| Contrôle | 96,6 | 0 | |
| Ozone | 251,4 | 160 | $$$ |
| Placébo | 204,0 | -19 | |
| Extrait selon l'invention à 3% | 176,6 | -30 | *** |

| | | | |
|---|---|---|---|
| *$$$ p*<*0,001 vs Contrôle* ^{∗∗} *0,001*<*p*<*0,01;* ^{∗∗∗} *p*<*0,001 vs Ozone* | | | |

### 4. Protection contre les effets délétères liés au soleil

Les Ultraviolets (UV) et les infrarouges (IR) pénètrent plus ou moins profondément dans la peau et sont responsables, entre autre, d'une diminution de sa fermeté et d'une augmentation de la quantité de radicaux libres relargués entrainant un vieillissement cutané prématuré. Ils sont également responsables de la formation des mélanomes et d'une immunodépression de la peau.

L'effet protecteur d'un extrait selon l'invention vis-à-vis d'un stress induit par les UV ou les IR a é été étudié.

### A- Protection contre les dommages à l'ADN induits par les UV (Comet Assay) :

Le maintien de l'intégrité nucléaire de la cellule, vis-à-vis des UVs, a été testé grâce à un test des comètes (Cornet Assay).

### Matériel et méthodes :

Des kératinocytes humains normaux ont été traités pendant 2h par un extrait selon l'invention, tel qu'obtenu dans l'exemple 1, à 0,001% (p/v).

Un Cornet Assay est réalisé suivant la méthode décrite par "Singh and al" en 1988 et par "De Meo and al" en 1991, et qui consiste en une irradiation des cellules par de la lumière à 4,5J/cm² (0,28J/cm² d'UVA ; 0,08J/cm² d'UVB et 4,14J/cm² de lumière visible) correspondant à une exposition de 1 à 3 min au soleil en plein été. Puis en une migration éléctrophorétique en gel d'agarose des ADN sur un gel.

Une valeur relative de χ² OTM est calculée grâce au logiciel "Systat Software", celle-ci étant directement proportionnel à la taille de la comète et donc au degré de protection de l'actif vis-à-vis des UV.

La significativité des résultats a été vérifiée par une analyse de variance sur le logiciel SigmaPlot (version 11.0, Systat Software, Chicago, IL, USA).

### Résultats et conclusion :

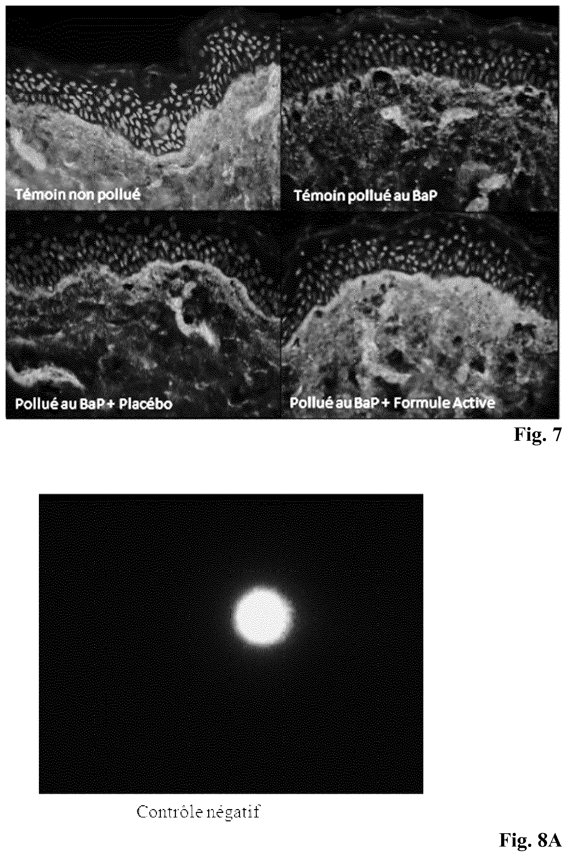

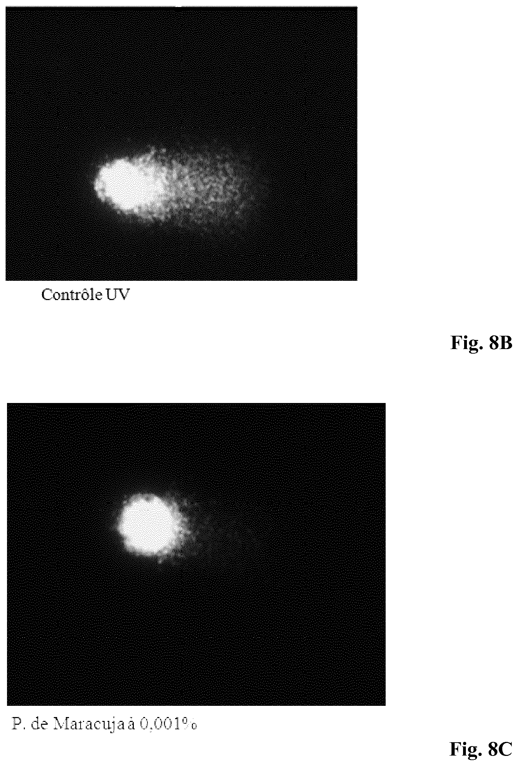

Les résultats sont présentés dans le tableau 15 et par la Figure 8.

Ces résultats montrent que l'extrait selon l'invention à 0,001%, a protégé les kératinocytes vis-à-vis des dommages à l'ADN induits par une irradiation UV (54% de protection, p<0,001).

L'extrait selon l'invention a donc une activité antioxydante, anti-radicalaire, anti-pollution et anti-âge.

**Tableau 15 - Pourcentage de protection des cellules vis-à-vis des UV**

| | **χ² OTM** | **% de Protection** | |
|---|---|---|---|
| Témoin sans UV | 2,09 ± 0,1 | 100 | |
| Témoin Irradié à 4,5J/cm² | 11.59 ± 0.41 | 0 | *** |
| **Extrait selon l'invention** à 0,001% | 6.43 ± 0.28 | 54.4 | *** |

| | | | |
|---|---|---|---|
| ^{∗∗∗} *p<0,001 vs cellules non irradié et vs cellules irradié à 4,5J*/*cm². Statistiques sur SigmaPlot* | | | |

### B- Inhibition de la production de MMP1 induite par les IR

Les Infrarouge (IR), qui représentent plus de la moitié du spectre solaire, peuvent induire la dégradation de la matrice dermique, contribuant au vieillissement cutané, en stimulant la production de protéases telles que la MMP1.

La capacité d'un extrait selon l'invention à protéger les cellules dermiques des effets néfastes des IR a été étudiée en évaluant la production de MMP1.

### Matériel et méthodes :

Des Fibroblastes Humains Normaux (FHN) ont été incubés en présence d'un extrait selon l'invention, tel qu'obtenu dans l'exemple 1, à 0,001% ou par de la déxaméthasone à 10⁻⁷M (référence antiinflammatoire) pendant 48h à la suite d'une irradiation de 1h par des infrarouges (0,57kJ/cm²).

Après incubation, les surnageants sont récupérés afin de doser la MMP1 (Kit ELISA, R&D Systems) libérée.

La significativité des résultats a été vérifiée par un test t de Student.

### Résultats et conclusion :

Les résultats sont présentés dans le tableau 16. Ces résultats montrent que l'extrait selon l'invention à 0,001%, a significativement inhibé la production de MMP1 induite par les Infra Rouges dans des Fibroblastes humains normaux.

L'extrait selon l'invention a donc une activité antioxydante, anti-radicalaire, anti-pollution et anti-âge.

**Tableau 16 - Production de MMP1 induite par les IR dans des fibroblastes humains normaux**

| | MMP1 (ng/ml) | % de Protection | |
|---|---|---|---|
| Témoin sans IR | 11 | 100 | *** |
| Témoin Irradié à 0,57kJ/cm² | 88.6 | 0 | |
| Déxaméthasone à 10⁻⁷M | 9.3 | 102 | *** |
| **rait selon l'invention** à 0,001%7 | 77.4 | 14 | * |

| | | | |
|---|---|---|---|
| ^{∗} p<*0,05 ;* ^{∗∗∗} *p*<0,*001 vs cellules irradiées* | | | |

### C- Protection contre le stress oxydant induit par les UVs :

Le potentiel antioxydant d'un extrait selon l'invention vis-à-vis de l'induction d'espèces réactives de l'oxygène (ERO) induit par une irradiation UV a été étudié.

### Matériel et méthodes :

Des kératinocytes humains normaux ont été incubés, pendant 24 heures, en présence d'un extrait selon l'invention, tel qu'obtenu dans l'exemple 1 à 0,001% et 0,005% (p/v) ou de la vitamine C à 500µM (Antioxydant de référence), avant incorporation de la sonde H2DCF-DA (incubation de 1h).

Les kératinocytes ont ensuite été stimulés par des UVs à 2400J/m² (2000J/m² en UVB et 400J/m² en UVA) puis remis en culture dans en présence d'un extrait selon l'invention, tel qu'obtenu dans l'exemple 1 à 0,001% et 0,005% (p/v) ou de la vitamine C à 500µM pendant 15min à 37°C.

La production d'ERO (Espèces Réactives de l'Oxygène) a été évaluée par mesure de fluorescence et la valeur obtenue a été normalisée par rapport à la quantité de cellules obtenue par une analyse de la viabilité cellulaire au MTT.

Les résultats ont été analysés statistiquement par une ANOVA à un facteur suivie d'un test de Tukey (Logiciel GraphPad Prism version 5.02, GraphPad Software, San Diego California USA).

### Résultats et conclusion :

L'extrait selon l'invention a significativement inhibé la production d'ERO par des kératinocytes en réponse à un stress oxydant induit par les UVs à 2400J/m² (Tableau 17).

**Tableau 17 - Production d'ERO dans des kératinocytes traités par des UVs à 2400J/m²**

| | **ERO (Intensité de fluorescence/MTT)** | **% d'évolution** | **Significativité** |
|---|---|---|---|
| Cellules contrôles | 30936 ± 4925 | | |
| Cellules irradiées (UV 2400J/m²) | 43038 ± 5735 | 39 | $$$ |
| Référence (Vitamine C) | 26005 ± 3723 | -40 | *** |
| Extrait selon l'invention à 0,001% | 22564 ±3849 | -48 | *** |
| Extrait selon l'invention à 0,005% | 19614 ± 4576 | -54 | *** |

| | | | |
|---|---|---|---|
| *$$$ p*<*0,001 vs témoin sans UVs* ^{∗∗∗} *p*<*0,001 vs témoin avec UVs* *ANOVA à un facteur suivie d'un test de Tukey* | | | |

### 5. Protection contre les effets d'un stress chimique

### Effet sur la production de PGE2 induit par le PMA :

La protection antiinflammatoire d'un extrait selon l'invention vis-à-vis d'une molécule chimique, le PMA, a été étudiée grâce à l'analyse du relargage de la Prostaglandine 2 (PGE2)

### Matériel et méthodes :

Des kératinocytes humains normaux ont été prétraités par un extrait selon l'invention, tel qu'obtenu dans l'exemple 1, à 0,002% et 0,005% et par de l'Indométacine à 10⁻⁶M (témoin-anti inflammatoire de la voie des Prostaglandines) pendant 24h à 37°C, et ce afin de pouvoir mesurer un niveau de protection de l'actif vis-à-vis d'une inflammation au PMA (Phorbol 12-myristate 13-acetate) à 0,1µg/ml utilisé sur ces mêmes tapis cellulaire pendant 24h de plus.

A la fin du traitement les surnageant sont récoltés et un dosage de la Prostaglandine E2 (PGE2 ; RetD Systems) est réalisé. La coloration, proportionnelle à la quantité de PGE2, a été mesurée par lecture de la densité optique (DO) à 450nm.

La significativité des résultats a été vérifiée par un test t de Student.

### Résultats :

Les résultats sont présentés dans le tableau 18. Ces résultats montrent que l'extrait selon l'invention aux deux concentrations a significativement diminué le relargage de la PGE2 vis-à-vis d'une induction par le PMA à 0,1µg/ml.

L'extrait selon l'invention a donc une activité antioxydante, anti-radicalaire, anti-pollution et anti-âge.

**Tableau 18 - Production de PGE2 dans des kératinocytes traités par du PMA**

| | **PGE2 (pg/ml)** | **Inhibition** | |
|---|---|---|---|
| Cellules contrôles | 39 ± 0 | **100%** | ^{∗∗∗} |
| **PMA à 0,1µg/ml** | **113806** ± **11441** | **0%** | |
| **Indométacine à 10^{- 6}M** | **52 ± 8** | **100%** | ^{∗∗∗} |
| **Extrait selon l'invention à 0,002%** | **35750 ± 2192** | **69%** | ^{∗∗} |
| **Extrait selon l'invention à 0,005%** | **30382 ± 2401** | **73%** | ^{∗∗} |

| | | | |
|---|---|---|---|
| ^{∗∗} *0,001<p<0,01 et* ^{∗∗∗} *p*<*0,001 vs cellules stimulées le PMA test t de Student* | | | |

### 6. Protection contre les effets du vieillissement

L'action répétée de stress environnementaux, tels que, la pollution, les effets néfastes du soleil, les molécules chimiques et toutes autres formes d'inductions d'un stress oxydant, entraine une dégradation de la matrice dermique et donc un vieillissement cutané prématuré.

Un extrait selon l'invention a été analysé sur un modèle de vieillissement cellulaire afin d'analyser leurs actions vis-à-vis de protéines qui sont sous-exprimées ou surexprimées avec l'âge.

### Matériel et méthodes :

Des kératinocytes sont mis en culture, et trypsinés toutes les semaines, pendant 4 semaines dans un milieu de culture induisant un phénotype de vieillissement (milieu « pro-âge ») en présence ou en absence d'un extrait selon l'invention, tel qu'obtenu dans l'exemple 1, à 0,000025% de MS.

Au bout de 3 semaines de cultures/passages une analyse protéomique est réalisée. Celles-ci étant impliquées dans divers voies cellulaires regroupées ensuite en 6 domaines : métabolisme, apoptose, détoxification, catabolisation protéique et synthèse protéique.

### Résultats et conclusion :

Les résultats sont présentés dans le tableau 19. Dans ce contexte d'induction du vieillissement, l'extrait selon l'invention a stimulé et/ou protégé l'expression de plusieurs protéines impliquées principalement dans la détoxification/protection cellulaire et dans les défenses immunitaires :

### Détoxification et protection cellulaire :

- **Stimulation de la Peroxiredoxine 2 (PRDX2)** : enzyme antioxydante jouant un rôle dans la protection cellulaire contre les dommages causés par les ERO intracellulaire.
- **Stimulation de la Carbonyl Reductase 1 (CBR1)** : dehydrogénase/réductase permettant de réduire les composés carbonylés (médicaments) et intervient dans la détoxification lors de la peroxydation lipidique. Celle-ci est inhibée dans les conditions « pro-âges » et stimulée par l'extrait selon l'invention dans ces conditions.
- **Inhibition de l'Aldehyde Dehydrogenase 2 (ALDH2)** : enzyme mitochondriale jouant un rôle dans la protection cellulaires et dans la différentiation qui vont catalyser/détoxifier des molécules aldéhydes/carbonyles (médicaments, pollution...). Cette protéine est stimulée dans les conditions « pro-âge », l'extrait selon l'invention restaure son niveau d'expression à un niveau basal.
- **Stimulation de la Fatty Acid Binding Protein 5 (FABP5)** : protéine chaperonne essentiellement exprimée dans l'épiderme et impliquée dans la régulation de l'homéostasie lipidique et donc jouant un rôle dans la fonction barrière. Les conditions « pro-âge » inhibent son expression, celle-ci est restaurée par les polyphenols de Maracuja.
- **Stimulation des Proteasome subunits β2 et β6 (PSMB2 et PSMB6)** : Le Proteasome est impliqué dans l'élimination des protéines altérées/oxydées et dans le renouvellement des protéines intracellulaires. Il est constitué de sous unités α et β qui vont, entre autre, couper les protéines altérées au niveau de la glutamine (β6) et au niveau trypsine (β2). L'âge diminue l'activité du Proteasome ce qui entraine une accumulation des protéines altérées/oxydées ; cet effet de l'âge est retrouvé dans les conditions « pro-âge » et dans ces conditions l'extrait selon l'invention stimule l'expression de ces deux sous-unités du protéasome.

### Défense immunitaire :

- **Inhibition de la Beta-2-microglobuline (B2MG):** petite protéine de surface (épiderme) impliquée dans la réponse immunitaire. Elle fait partie du complexe majeur d'Histocompatibilité et est surexprimée en condition pathologique ce qui va induire la production d'interleukines, notamment 6 et 8, ainsi que la 10 qui est une interleukine immunosuppressive. Ici, dans les conditions induisant un vieillissement, son expression est augmentée. L'extrait selon l'invention empêche cette augmentation.
- **Inhibition de la Lectin, mannose-binding, 1 (LMAN1)** : protéine faisant partie de la réponse immunitaire inné en permettant la phagocytose des cellules apoptotiques et des pathogènes. Une déficience en ce gène implique une augmentation des débris cellulaires dans la peau. Il n'est pas ou peu présent à l'état basal et est augmenté en condition inflammatoire (Ex : UV). Dans les conditions « pro-âge », son expression est augmentée et modulée sous l'action de l'extrait selon l'invention.

**Tableau 19 - Expression protéiques de marqueurs impliqués dans la détoxification et dans l'immunité (en %)**

| | **% d'induction vis-à-vis du Milieu normal** | | **% d'induction vis-à-vis du milieu « Pro-Age »** | |
|---|---|---|---|---|
| **Protéines** | **Milieu** « **Pro-Age** » | **Extrait selon l'invention 0,000025%** | **Milieu** « **Pro-Age** » | **Extrait selon l'invention 0,000025%** |
| **Peroxiredoxin 2 (PRDX2)** | 11.46 | 16.68 | 0.00 | 45.51 |
| **Carbonyl reductase 1 (CBR1)** | -15.87 | -7.46 | 0.00 | 52.99 |
| **Aldéhyde dehydrogenase 2 (ALDH2)** | 46.81 | 4.97 | 0.00 | -89.38 |
| **Fatty acid binding protein 5 (FABP5)** | -24.82 | 4.68 | 0.00 | 118.87 |
| **Proteasome β2 (PSMB2)** | -21.15 | -10.96 | 0.00 | 48.21 |
| **Proteasome β6 (PSMB6)** | -16.85 | -10.92 | 0.00 | 35.21 |
| **Beta-2-microglobulin (B2MG)** | 31.16 | -0.57 | 0.00 | -101.83 |
| **Lectin, mannose-binding, 1 (LMAN1)** | 40.13 | 3.71 | 0.00 | -90.75 |

### 7. Conclusion

Ces différents tests montrent un effet anti-inflammatoire, anti-oxydant, et anti-pollution et donc anti-âge de l'extrait polyphénolique selon l'invention.

### Exemple 4 : évaluation de l'efficacité in vivo de l'actif polyphénols de passiflore versus placebo par mesure de la quantité deMDA, SOD, catalase (CAT) et de protéines carbonylées

### Design de l'étude :

- Etude en double aveugle.
- Etude comparative, randomisée

### Population :

Deux groupes de 30 sujets (1 groupe Actif et 1 groupe Placebo), de sexe féminin, entre 30 et 50 ans, Asiatiques, tous types de peau, vivant en environnement pollué.

### Modalités d'utilisation :

Applications des produits sur le visage réalisées par les sujets eux-mêmes en hémi visage, à leur domicile, 2 fois par jour (matin et soir) pendant 28 jours.

### Déroulement de l'étude :

**Tableau 20 - déroulement de l'étude clinique**

| **Evaluation** | **T0** | **T 28jours** |
|---|---|---|
| Prélèvement de cheveux | X | X |
| Prélèvement biologique au niveau des joues pour MDA, CAT, SOD | X | X |
| Prélèvement biologique au niveau des joues pour Protéines carbonylées | X | X |

### Age moyen réel par panel :

- Panel total : 39.9 ans (entre 30 et 50 ans)

### Composition administrée :

### Formule Placebo

| **Nom Matière** | **INCI UE** | **% Matière** |
|---|---|---|
| EAU PURIFIEE R&D | AQUA | 75,12 |
| EDETATEDESODIUM | DISODIUM EDTA | 0,10 |
| BUTYLENEGLYCOL | BUTYLENE GLYCOL | 3,00 |
| CARBOPOLULTREZ10 | CARBOMER | 0,60 |
| OCTANEDIOLXI | CAPRYLYL GLYCOL | 0,30 |
| EMULIUMDELTA | CETYL ALCOHOL | 3,50 |
| | GLYCERYL STEARATE | |
| | PEG-75 STEARATE | |
| | CETETH-20 | |
| | STEARETH-20 | |
| ISONONYLISONONANOAT | ISONONYL ISONONANOATE | 10,00 |
| ALCOOLCETYLIQUEPUR | CETYL ALCOHOL | 0,50 |
| CAPRYLOYLGLYCINE | CAPRYLOYL GLYCINE | 0,80 |
| LESSIVESOUDEXI | AQUA | 1,08 |
| | SODIUM HYDROXIDE | |
| EAU PURIFIEE R&D | AQUA | 2,00 |
| EAU PURIFIEE R&D | AQUA | 3,00 |
| | | **100,00** |

### Formule Actif

| **Nom Matière** | **INCI UE** | **% Matière** |
|---|---|---|
| PLACEBO | | 97 |
| | AQUA | 3 |
| POLYPHENOLS DE MARACUJA | PROPANEDIOL | |
| 1KG | PASSIFLORA EDULIS FRUIT | |
| | EXTRACT | |
| | | **100** |

### 1. Evaluation de la pollution reçue par le sujet à T0 et T28

A T0 et T28, une mèche de cheveux de 1 cm à partir du cuir chevelu a été prélevée. Une analyse biochimique a été réalisée afin de déterminer l'exposition aux métaux lourds des sujets à chaque temps. L'analyse a porté sur 10 sujets, 5 de chaque panel.

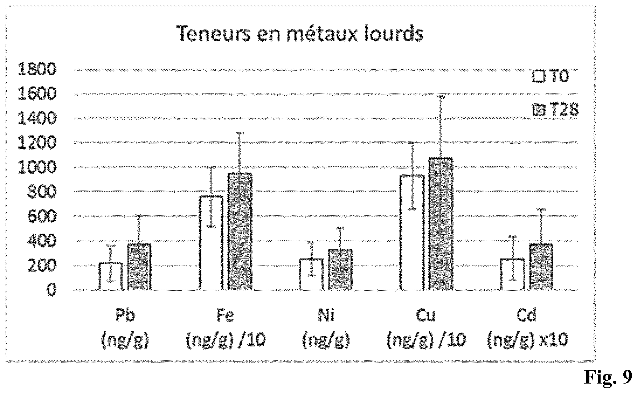

Les résultats (Tableau 21 et Figure 9) montrent une quantité constante de métaux lourds ou une légère augmentation. On peut conclure que l'exposition des sujets à la pollution n'a pas diminuée lors de l'étude, écartant l'hypothèse d'un effet bénéfique dû uniquement à une variation de pollution.

**Tableau 21 - Teneurs en métaux lourds**

| | **Pb (ng/g)** | **Fe (ng/g)** | **Ni (ng/g)** | **Cu (ng/g)** | **Cd (ng/g)** |
|---|---|---|---|---|---|
| **T0** | 218,5 | 7603,9 | 249,7 | 9283,8 | 25,5 |
| **T28** | 366 | 9487 | 328 | 10711 | 37 |
| **Différence** | Oui | Oui | Non | Non | Oui |
| **Significative** | p=0.0098 | p=0.0488 | p=0.0840 | p=0.2324 | p=0.0019 |

### 2. Evaluation de la quantité de MDA, CAT et SOD à T0 et T28

A T0 et T28, un écouvillon sur chaque joue des sujets a été prélevé. Une analyse biochimique a été réalisée afin de déterminer la quantité de MDA, CAT et SOD. L'analyse a porté sur 30 sujets, 15 de chaque panel.

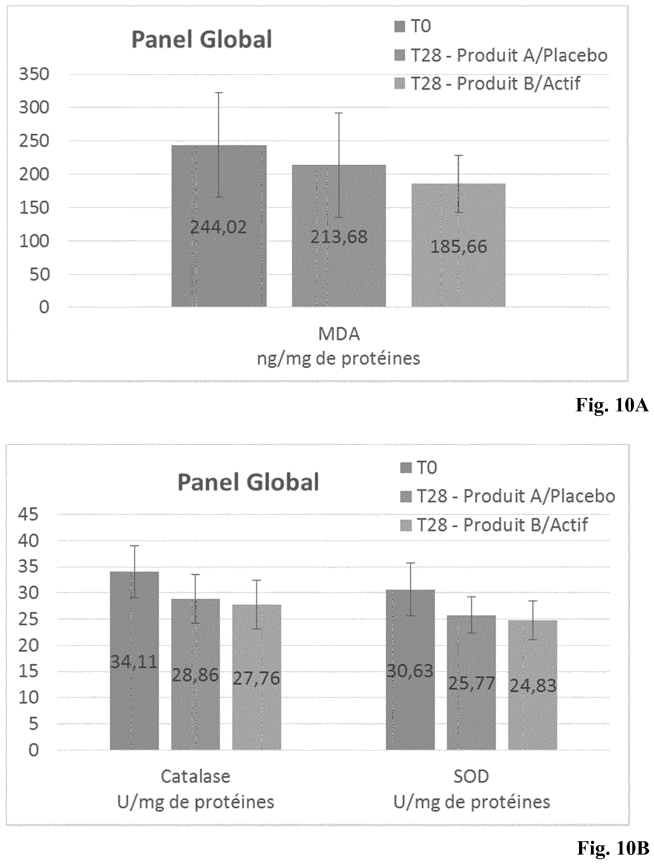

La quantité de MDA, CAT et SOD diminue significativement entre T0 et T28 pour l'actif et le placebo, avec une diminution significativement plus importante pour l'actif (Tableau 22 et Figures 10A et 10B).

**Tableau 22 - Teneurs en MDA, CAT et SOD à T0 et T28**

| | **MDA** | | **CAT** | | **SOD** | |
|---|---|---|---|---|---|---|
| | **Placebo** | **Actif** | **Placebo** | **Actif** | **Placebo** | **Actif** |
| **T0** | 244.0 ±77.9 | 244.0 ±77.9 | 34.1 ±4.98 | 34.1 ±4.98 | 30.6 ±5.0 | 30.6 ±5.0 |
| **T28** | 213.7 ±65.1 | 185.7 ±56.3 | 28.9±4.7 | 27.8 ±4.6 | 25.8 ±3.4 | 24.8 ±3.7 |
| **% évolution** | -12.4% | -23.9% | -15.4% | -18.6% | -15.9% | -18.9% |
| **Significativité** | Oui | Oui | Oui | Oui | Oui | Oui |
| **T28-T0** | p<0.01 | p<0.01 | p<0.01 | p<0.01 | p<0.01 | p<0.01 |
| **Significativité** | Oui | | Oui | | Oui | |
| **Placebo vs Actif** | p<0.01 | | p<0.01 | | p<0.01 | |

### 3. Evaluation de la quantité de protéines carbonylées à T0 et T28

A T0 et T28, un D-squame sur chaque joue des sujets a été prélevé. Un marquage biochimique a été réalisé afin de déterminer la quantité de protéines carbonylées. L'analyse a porté sur 20 sujets, 10 de chaque panel.

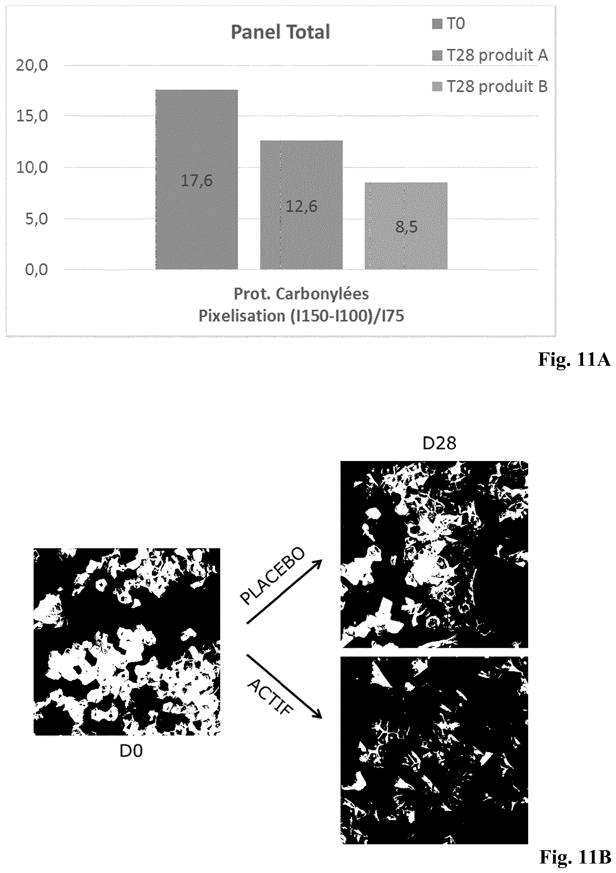

La quantité de protéines carbonylées diminue significativement entre T0 et T28 pour l'actif et le placebo, avec une diminution significativement plus importante pour l'actif (Tableau 23 et Figures 11A et 11B).

**Tableau 23 - Teneurs en protéines carbonylées à T0 et T28**

| | **Protéines carbonylées** | |
|---|---|---|
| | **Placebo** | **Actif** |
| **T0** | 17.6 ±5.6 | 17.6 ±5.6 |
| **T28** | 12.6 ±7.9 | 8.5 ±5.5 |
| **% évolution** | -28.1% | -51.5% |
| **Significativité T28-T0** | Oui | Oui |
| | p<0.01 | p<0.01 |
| **Significativité Placebo vs Actif** | Oui | |
| | p=0.01 | |

### 4. Synthèse des mesures biochimiques

Les résultats démontrent une efficacité anti radicalaire/détoxifiante aussi bien sur le panel total que sur les sous panels non-fumeurs et fumeurs.

L'actif permet de se substituer aux défenses naturelles de la peau que sont SOD et CAT, conduisant ainsi à une quantité de résidus issus du processus de détoxification (MDA) moindre.

Les résultats des protéines carbonylées confirment cette action anti radicalaire/détoxifiante.

### 5. Résumé de l'efficacité

L'actif (3%) a démontré une efficacité significative sur les paramètres suivants :

### • Quantité de MDA au niveau de la joue

L'actif a induit les effets suivants sur la quantité de MDA mesurée à partir de prélèvement biologique au niveau de la joue:
- Sur le panel global (30 sujets) une baisse significative de 23.9%. L'effet observé est significatif en comparaison de l'effet observé avec le Placebo.

### • Quantité de SOD au niveau de la joue

L'actif a induit les effets suivants sur la quantité de SOD mesurée à partir de prélèvement biologique au niveau de la joue:
- Sur le panel global (30 sujets) une baisse significative de 18.6%. L'effet observé est significatif en comparaison de l'effet observé avec le Placebo.

### • Quantité de CAT au niveau de la joue

L'actif a induit les effets suivants sur la quantité de CAT mesurée à partir de prélèvement biologique au niveau de la joue:
- Sur le panel global (30 sujets) une baisse significative de 18.9%. L'effet observé est significatif en comparaison de l'effet observé avec le Placebo.

### • Quantité de protéines carbonylées au niveau de la joue

L'actif a induit les effets suivants sur la quantité de protéines carbonylées mesurée à partir de prélèvement biologique au niveau de la joue:
- Sur le panel global (20 sujets) une baisse significative de 51.5%. L'effet observé est significatif en comparaison de l'effet observé avec le Placebo.
   **→** L'ensemble de ces résultats démontre des effets significatifs d'un point de vue détoxifiant.

### Exemple 5 : Compositions pour application par voie topique

Plusieurs compositions pour application par voie topique sont présentées ci-dessous. L'extrait polyphénolique de graines de Passiflore, de l'exemple 1 ou 2, peut être incorporé à divers produits cosmétiques, tels que des eaux nettoyantes, des émulsions huile dans eau, des émulsions eau dans huile, des huiles, des laits, des lotions, des shampooings, des produits moussants et sprays, dont les compositions sont présentées ci-dessous à titre d'exemples.

### EAU NETTOYANTE PEAU SENSIBLE

| **Matière première / Nom commercial ou Nom INCI** | **%** |
|---|---|
| CAPRYLOYL GLYCINE | De 0 à 1 % |
| LESSIVE SOUDE | De 0 à 1 % |
| SEQUESTRANT | De 0 à 1 % |
| BUTYLENE GLYCOL | De 1 à 5 % |
| BETA CAROTENE | De 0 à 2 % |
| *EXTRAIT POLYPHENOLIQUE DE PASSIFLORE* | De 0,001 à 10 % |
| CONSERVATEURS | De 0 à 1 % |
| PEG-32 | De 1 à 5 % |
| PEG-7 PALMCOCOATE | De 1 à 5 % |
| GLUCONATE ZINC | De 0 à 1 % |
| ACIDE CITRIQUE | De 0 à 1 % |
| EAU PURIFIÉE | QSP 100 % |
| PARFUM | De 0 à 1 % |
| POLOXAMER 184 | De 1 à 5 % |

### EMULSION ANTI-AGE

| **Matière première** / **Nom commercial ou Nom INCI** | % |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1% |
| VITAMINE C | De 0 à 5% |
| *EXTRAIT POLYPHENOLIQUE DE PASSIFLORE* | De 0,01 à 10 % |
| ISONONYL ISONONANOATE | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

## Revendications

1. Extrait polyphénolique de graines de Passiflore, en particulier de graines de *Passiflora incarnata* ou de *Passiflora edulis,* comprenant au moins 30% en poids de polyphénols, exprimés en équivalent d'acide gallique, par rapport au poids de l'extrait sec, et au moins 10% en poids d'acides organiques, par rapport au poids de l'extrait sec, dans lequel lesdits acides organiques sont l'acide acétique, l'acide malique, l'acide citrique ou leurs mélanges.

2. Extrait selon la revendication 1, comprenant au moins 35%, avantageusement au moins 40%, en poids de polyphénols, exprimés en équivalent d'acide gallique, par rapport au poids de l'extrait sec.

3. Extrait selon la revendication 1 ou 2, dans lequel au moins 50% en poids desdits polyphénols sont des dérivés catéchiques, exprimés en équivalent d'acide gallique, par rapport au poids de polyphénols dans l'extrait sec.

4. Extrait selon l'une quelconque des revendications 1 à 3, **caractérisé en ce qu'**il est obtenu par extraction solide/liquide des graines de Passiflore dans un solvant choisi parmi l'eau, les glycérols, les glycols, et leurs mélanges.

5. Extrait selon l'une quelconque des revendications 1 à 4, **caractérisé en ce qu'**il est obtenu par extraction solide/liquide des graines de Passiflore dans un solvant choisi parmi les mélanges binaires eau/glycérol, eau/glycol, et leurs mélanges, avantageusement dans une proportion comprise entre 30 et 90% de glycérol et/ou de glycol dans l'eau.

6. Procédé de préparation d'un extrait polyphénolique des graines de passiflore tel que défini selon l'une quelconque des revendications 1 à 5, ledit procédé comprenant au moins une étape d'extraction solide/liquide dans un solvant choisi parmi l'eau, les glycérols, les glycols, et leurs mélanges.

7. Procédé selon la revendication 6, **caractérisé en ce qu'**il comprend les étapes successives suivantes :
a) broyage des graines ;
b) éventuellement délipidation des graines, de préférence par pressage, par extraction éthanolique ou par extraction CO₂ supercritique ;
c) extraction solide/liquide des graines broyées et éventuellement délipidées dans un solvant choisi parmi l'eau, les glycérols, les glycols, et leurs mélange ;
d) séparation de la phase solide et de la phase liquide par décantation, et/ou centrifugation et/ou filtrations successives ; et
e) éventuellement séchage de l'extrait obtenu à l'étape d).

8. Procédé selon la revendication 6 ou 7, **caractérisé en ce que** ledit solvant d'extraction solide/liquide est choisi parmi les mélanges binaires eau/glycérol, eau/glycol, et leurs mélanges, avantageusement dans une proportion comprise entre 40 % et 90%, de préférence entre 50 % et 90 %, plus préférentiellement entre 60 % et 80 %, notamment 70 %, de glycérol et/ou de glycol dans l'eau.

9. Composition comprenant en tant que principe actif, un extrait polyphénolique de graines de Passiflore tel que défini dans l'une quelconque des revendications 1 à 5 ou d'un extrait susceptible d'être obtenu par le procédé selon l'une quelconque des revendications 6 à 8, et avantageusement un excipient approprié.

10. Composition selon la revendication 9, comprenant de 0,001 à 10% en poids, avantageusement de 0,01 à 5% en poids, dudit extrait polyphénolique de graines de Passiflore, le poids de l'extrait étant exprimé en extrait sec, par rapport au poids total de la composition.

11. Extrait selon l'une quelconque des revendications 1 à 5 ou extrait susceptible d'être obtenu par le procédé selon l'une quelconque des revendications 6 à 8 ou composition selon l'une quelconque des revendications 9 et 10 pour son utilisation pour prévenir et/ou traiter :
- des troubles ou pathologies de la peau et/ou des muqueuses et/ou des phanères, avantageusement des réactions inflammatoires, des réactions d'oxydation, des désordres liés à des attaques radicalaires liées ou non à la pollution, des troubles de la barrière ou de l'homéostasie, et/ou
- des troubles vasculaires, et/ou
- des altérations du tissu adipeux
- le vieillissement, notamment le vieillissement actinique, de la peau et/ou des muqueuses et/ou des phanères.

12. Utilisation d'un extrait selon l'une quelconque des revendications 1 à 5 ou d'un extrait susceptible d'être obtenu par le procédé selon l'une quelconque des revendications 6 à 8 ou d'une composition selon l'une quelconque des revendications 9 et 10, pour prévenir et/ou traiter le vieillissement chronologique de la peau et/ou des muqueuses et/ou des phanères.

## Patentansprüche

1. Polyphenolextrakt aus Passionsblumensamen, vor allem aus Samen von *Passiflora incarnata* oder *Passiflora edulis,* umfassend mindestens 30 Gew.-% Polyphenole, ausgedrückt in Gallussäureäquivalent, im Verhältnis zum Gewicht des Trockenextrakts, und mindestens 10 Gew.-% organische Säuren im Verhältnis zum Gewicht des Trockenextrakts, wobei die organischen Säuren die Essigsäure, die Apfelsäure, die Zitronensäure oder deren Gemische sind.

2. Extrakt nach Anspruch 1, umfassend mindestens 35, in vorteilhafter Weise mindestens 40 Gew.-% Polyphenole, ausgedrückt in Gallussäureäquivalent, im Verhältnis zum Gewicht des Trockenextrakts.

3. Extrakt nach Anspruch 1 oder 2, wobei mindestens 50 Gew.-% der Polyphenole Catechinderivate sind, ausgedrückt in Gallussäureäquivalent, im Verhältnis zum Polyphenolgewicht im Trockenextrakt.

4. Extrakt nach einem der Ansprüche 1 bis 3, **dadurch gekennzeichnet, dass** er durch Fest-Flüssig-Extraktion der Passionsblumensamen in einem Lösungsmittel gewonnen wird, das aus Wasser, den Glycerolen, den Glycolen und deren Gemischen ausgewählt ist.

5. Extrakt nach einem der Ansprüche 1 bis 4, **dadurch gekennzeichnet, dass** er durch Fest-Flüssig-Extraktion der Passionsblumensamen in einem Lösungsmittel gewonnen wird, das aus den binären Gemischen Wasser/Glycerol, Wasser/Glycol und deren Gemischen gewonnen wird, in vorteilhafter Weise in einem Verhältnis zwischen 30 und 90% Glycerol und/oder Glycol in Wasser.

6. Verfahren zur Herstellung eines Polyphenolextrakts aus Passionsblumensamen nach einem der Ansprüche 1 bis 5, wobei das Verfahren mindestens einen Fest-Flüssig-Extraktionsschritt in einem Lösungsmittel umfasst, das aus Wasser, den Glycerolen, den Glycolen und deren Gemischen ausgewählt ist.

7. Verfahren nach Anspruch 6, **dadurch gekennzeichnet, dass** es die folgenden aufeinanderfolgenden Schritte umfasst:
a) Zerkleinern der Samen;
b) gegebenenfalls Delipidation der Samen, vorzugsweise durch Pressen, durch Ethanolextraktion oder durch superkritische CO₂-Extraktion;
c) Fest-Flüssig-Extraktion der zerkleinerten und gegebenenfalls delipidierten Samen in einem Lösungsmittel, das aus Wasser, den Glycerolen, den Glycolen und deren Gemischen ausgewählt ist;
d) Trennen der festen Phase und der flüssigen Phase durch Dekantieren und/oder Zentrifugieren und/oder aufeinanderfolgende Filtrationen; und
e) gegebenenfalls Trocknen des in Schritt d) enthaltenen Extrakts.

8. Verfahren nach Anspruch 6 oder 7, **dadurch gekennzeichnet, dass** das Fest-Flüssig-Extraktionslösungsmittel aus den binären Gemischen Wasser/Glycerol, Wasser/Glycol und deren Gemischen in vorteilhafter Weise in einem Verhältnis ausgewählt ist, das zwischen 40% und 90%, vorzugsweise zwischen 50% und 90%, vorzugsweiser zwischen 60% und 80%, insbesondere 70% Glycerol und/oder Glycol in Wasser, liegt.

9. Zusammensetzung, die als Wirkstoff einen Passionsblumensamen-Phenolextrakt nach einem der Ansprüche 1 bis 5 oder einen Extrakt umfasst, der nach dem Verfahren nach einem der Ansprüche 6 bis 8 gewinnbar ist, und in vorteilhafter Weise einen geeigneten Hilfsstoff.

10. Zusammensetzung nach Anspruch 9, umfassend 0,001 bis 10 Gew.-%, in vorteilhafter Weise 0,01 bis 5 Gew.-% des Passionsblumensamen-Phenolextrakts, wobei das Gewicht des Extrakts in Trockenextrakt im Verhältnis zum Gesamtgewicht der Zusammensetzung ausgedrückt ist.

11. Extrakt nach einem der Ansprüche 1 bis 5 oder Extrakt, der durch das Verfahren nach einem der Ansprüche 6 bis 8 gewinnbar ist oder Zusammensetzung nach einem der Ansprüche 9 und 10 für deren Verwendung zur Vorbeugung und/oder Behandlung:
- von Krankheiten oder Erkrankungen der Haut und/oder der Schleimhäute und/oder der Hautanhangsgebilde, in vorteilhafter Weise von entzündlichen Reaktionen, Oxidationsreaktionen, Störungen im Zusammenhang mit Angriffen durch Radikale im Zusammenhang mit Schadstoffen oder nicht, Krankheiten der Barriere oder der Homöostase, und/oder
- der Gefäßkrankheiten, und/oder
- der Beeinträchtigungen des Fettgewebes,
- der Alterung, insbesondere der aktinischen Alterung der Haut und/oder der Schleimhäute und/oder der Hautanhangsgebilde.

12. Verwendung eines Extrakts nach einem der Ansprüche 1 bis 5 oder eines Extrakts, der durch das Verfahren nach einem der Ansprüche 6 bis 8 gewinnbar ist oder einer Zusammensetzung nach einem der Ansprüche 9 und 10 zur Vorbeugung und/oder Behandlung der chronologischen Alterung der Haut und/der der Schleimhäute und/oder der Hautanhangsgebilde.

## Claims

1. A polyphenolic extract of passionflower seeds, in particular Passiflora incarnata or Passiflora edulis seeds, comprising at least 30 percent by weight polyphenols, expressed as gallic acid equivalent, relative to the weight of the dry extract, and at least 10 percent by weight organic acids, relative to the weight of the dry extract, wherein said organic acids are acetic acid, malic acid, citric acid or mixtures thereof.

2. The extract according to claim 1, comprising at least 35 percent, advantageously at least 40 percent, by weight polyphenols, expressed as gallic acid equivalent, relative to the weight of the dry extract.

3. The extract according to claim 1 or 2, wherein at least 50 percent by weight of said polyphenols are catechol derivatives, expressed as gallic acid equivalent, relative to the weight of polyphenols in the dry extract.

4. The extract according to any one of claims 1 to 3, **characterized in that** it is obtained by solid/liquid extraction of passionflower seeds in a solvent selected from water, glycerols, glycols, and mixtures thereof.

5. The extract according to any one of claims 1 to 4, **characterized in that** it is obtained by solid/liquid extraction of passionflower seeds in a solvent selected from binary mixtures water/glycerol, water/glycol, and mixtures thereof, advantageously in a proportion comprised between 30 and 90 percent glycerol and/or glycol in water.

6. A method for the preparation of a polyphenolic extract of passionflower seeds as defined according to any one of claims 1 to 5, said method comprising at least one solid/liquid extraction step in a solvent selected from water, glycerols, glycols, and mixtures thereof.

7. The method according to claim 6, **characterized in that** it comprises the following successive steps:
a) grinding of the seeds;
b) optionally defatting of the seeds, preferably by pressing, by ethanolic extraction or by supercritical CO₂ extraction;
c) solid/liquid extraction of the ground and optionally defatted seeds in a solvent selected from water, glycerols, glycols, and mixtures thereof;
d) separation of the solid phase and the liquid phase by decantation, and/or centrifugation and/or successive filtrations; and
e) optionally drying of the extract obtained in step d) .

8. The method according to claim 6 or 7, **characterized in that** said solid/liquid extraction solvent is selected from binary mixtures water/glycerol, water/glycol, and mixtures thereof, advantageously in a proportion comprised between 40 percent and 90 percent, preferably between 50 percent and 90 percent, more preferably between 60 percent and 80 percent, in particular 70 percent, of glycerol and/or glycol in water.

9. A composition comprising, as active ingredient, a polyphenolic extract of passionflower seeds as defined in any one of claims 1 to 5 or an extract obtainable by the method according to any one of claims 6 to 8, and advantageously a suitable excipient.

10. The composition according to claim 9, comprising from 0.001 to 10 percent by weight, advantageously from 0.01 to 5 percent by weight, of said polyphenolic extract of passionflower seeds, the weight of the extract being expressed as dry extract, relative to the total weight of the composition.

11. An extract according to any one of claims 1 to 5 or extract obtainable by the method according to any one of claims 6 to 8 or composition according to any one of claims 9 and 10 for its use in preventing and/or treating:
- disorders or pathologies of the skin and/or mucous membranes and/or skin appendages, advantageously inflammatory reactions, oxidation reactions, disorders related to radical attacks related or not to pollution, disorders of the barrier or of homeostasis, and/or
- vascular disorders, and/or
- alterations in adipose tissue
- aging, in particular actinic aging, of the skin and/or of the mucous membranes and/or of the skin appendages.

12. A use of an extract according to any one of claims 1 to 5 or of an extract obtainable by the method according to any one of claims 6 to 8 or of a composition according to any one of claims 9 and 10, for preventing and/or treating chronological aging of the skin and/or mucous membranes and/or skin appendages.
